# EUROPÄISCHE PATENTANMELDUNG

(11) **EP 4 811 630 A1**
(43) Veröffentlichungstag der Anmeldung: **23.09.2026**
(21) Anmeldenummer: 25165446.3
(22) Anmeldetag: 21.03.2025
(51) Int. Cl.: H02K 21/24, H02K 41/03, A61M 60/00, H02K 7/09, H02K 1/14

(54) **ELEKTRISCHE BAUGRUPPE, STEUERVERFAHREN, STEUEREINHEIT UND ELEKTRISCHES SYSTEM**

(71) Anmelder: Berlin Heart GmbH, 12247 Berlin (DE); Peters, Oliver, 14129 Berlin (DE)
(72) Erfinder: PETERS, Oliver, 14129 Berlin (DE)
(74) Vertreter: Pfenning, Meinig & Partner mbB

(57) **Zusammenfassung**

Die Anmeldung betrifft eine elektrische Baugruppe (1100), ein Steuerverfahren, eine Steuereinheit (700) und ein elektrisches System. Die Baugruppe (1100) umfasst eine stationäre Komponente (1200), umfassend eine Mehrzahl von Spuleneinheiten (1210), wobei jede der Mehrzahl von Spuleneinheiten (1210) zum Erzeugen eines variablen Magnetfelds ansteuerbar ist, eine aktuierbare Komponente (1300), die relativ zu der stationären Komponente (1200) beweglich ist und eine Magnetanordnung (1310) umfasst, wobei jede einzelne der Mehrzahl von Spuleneinheiten (1210) zum Erzeugen des jeweiligen variablen Magnetfelds derart eingerichtet ist, dass durch Wechselwirkung des jeweiligen variablen Magnetfelds mit der Magnetanordnung (1310) Kräfte und/oder Momente bezüglich mehrerer Freiheitsgrade auf die aktuierbare Komponente (1300) wirken, so dass durch Ansteuern einer beliebigen Kombination von höchstens sechs der Mehrzahl von Spuleneinheiten (1210) eine Position und/oder Bewegung der aktuierbaren Komponente (1300) relativ zu der stationären Komponente (1200) in sechs linear unabhängigen Freiheitsgraden steuerbar ist.

## Beschreibung

Die Anmeldung betrifft eine elektrische Baugruppe, ein Steuerverfahren für eine elektrische Baugruppe, eine Steuereinheit für eine elektrische Baugruppe und ein elektrisches System mit einer elektrischen Baugruppe.

Der Anmeldungsgegenstand ist mit besonderem Vorteil im Bereich von Pumpsystemen, insbesondere Systemen für die Herzunterstützung und/oder zur Unterstützung einer Fontanzirkulation, einsetzbar. Auch der Einsatz für Kraftstoffpumpen, Vakuumpumpen, Umwälzpumpen und andere Arten von Pumpensystemen ist möglich.

Auch außerhalb des Bereichs der Pumpsysteme ergeben sich zahlreiche Verwendungsmöglichkeiten für den Anmeldungsgegenstand. So kann dieser etwa bei Elektromotoren, Lüftern, Kompressoren, Mischern, Turbinen, Zentrifugen, Lageregelungs- und Stabilisationssystemen (etwa Reaktionsrädern, Stabilisatoren für optische Abbildungssysteme etc.) und in anderen Bereichen verwendet werden.

Den genannten Systemen ist gemein, dass sie eine elektrische Baugruppe mit einer stationären Komponente und einer gegenüber der stationären Komponente beweglichen aktuierbaren Komponente umfassen, wobei ein Aktuieren der aktuierbaren Komponente mittels magnetischer Wechselwirkung der stationären Komponente und der aktuierbaren Komponente erfolgt. So kann die stationäre Komponente beispielsweise der Stator eines Elektromotors, die aktuierbare Komponente der Rotor des Elektromotors sein (mit entsprechenden Anwendungen für Pumpen, Lüfter, Kompressoren, Mischer, Turbinen, Zentrifugen und andere elektromotorisch angetriebene Systeme).

Grundsätzlich bestehen für eine relative Position und/oder Bewegung der stationären Komponente und der aktuierbaren Komponente sechs linear unabhängige Freiheitsgrade (insbesondere als drei rotatorische und drei translatorische Freiheitsgrade bezüglich dreier orthogonaler kartesischer Achsen darstellbar, aber auch eine abweichende Wahl bzw. Darstellung linear unabhängiger Freiheitsgrade ist möglich).

Es ist in vielen Fällen wünschenswert oder erforderlich, dass magnetische Kräfte bezüglich aller sechs Freiheitsgrade zwischen der stationären Komponente und der aktuierbaren Komponente wirken können. Aus dem Stand der Technik sind hierfür verschiedene Ansätze bekannt, etwa kann einfach jeder der Freiheitsgrade mittels eines separaten Aktors (beispielsweise Spule eines Hubmagneten oder Motorphase eines Elektromotors) angesteuert werden. Bei diesem Ansatz wird allerdings der Bauraum weder im Stator noch im Rotor effizient genutzt. Zudem ist der Wirkungsgrad potentiell gering, da zum Erzeugen einer großen Aktorkraft in einem einzelnen Freiheitsgrad nur ein Sechstel der magnetischen Hardware genutzt wird und die verbleibenden Komponenten Ballast bilden oder den Spulenquerschnitt des aktiven Aktors einschränken. Jedoch ist eine kompakte Bauform ebenso wünschenswert wie ein effizienter Betrieb und eine hohe Standzeit.

Bei bestimmten Anwendungen, beispielsweise bei Herzunterstützungssystemen oder sicherheitskritischen Systemen, kann zudem eine Redundanz der Aktoren hinsichtlich der Eigenschaft, alle Freiheitsgrade ansteuern zu können, erwünscht sein, um hinreichende Sicherheit im Betrieb, insbesondere mindestens Erstfehlersicherheit (etwa hinsichtlich des Ausfalls eines ersten Aktors und/oder damit verbundener Komponenten), herstellen zu können, was wiederum zu Lasten der Kompaktheit und/oder Effizienz gehen kann. Im vorbeschriebenen Beispiel etwa wären hierzu bereits zwölf Aktoren erforderlich, um jeden einzelnen Aktor redundant auszuführen.

Vor diesem Hintergrund liegt der Anmeldung die Aufgabe zugrunde, Lösungen hinsichtlich der magnetischen Lagerung und/oder Ansteuerung einer aktuierbaren Komponente relativ zu einer stationären Komponente bereitzustellen, die die genannten Anforderungen wenigstens zum Teil erfüllen bzw. ein geeignetes Gleichgewicht der gewünschten Eigenschaften bereitstellen und/oder die genannten Nachteile wenigstens zum Teil vermeiden oder verringern.

Zur Lösung der Aufgabe werden die Gegenstände der unabhängigen Ansprüche vorgeschlagen. Bevorzugte Ausgestaltungen und optionale Weiterbildungen ergeben sich mit den Merkmalen der abhängigen Ansprüche.

Eine offenbarte elektrische Baugruppe (im Folgenden mitunter auch als Maschine bezeichnet) umfasst eine stationäre Komponente, umfassend eine Mehrzahl von Spuleneinheiten, wobei jede der Mehrzahl von Spuleneinheiten zum Erzeugen eines variablen Magnetfelds ansteuerbar ist, und eine aktuierbare Komponente, die relativ zu der stationären Komponente beweglich ist und eine Magnetanordnung umfasst.

Vorzugsweise ist vorgesehen, dass jede einzelne der Mehrzahl von Spuleneinheiten zum Erzeugen des jeweiligen variablen Magnetfelds derart eingerichtet ist, dass durch Wechselwirkung des jeweiligen variablen Magnetfelds mit der Magnetanordnung Kräfte und/oder Momente bezüglich mehrerer Freiheitsgrade auf die aktuierbare Komponente wirken, und zwar insbesondere so, dass durch Ansteuern einer beliebigen Kombination von höchstens sechs der Mehrzahl von Spuleneinheiten eine Position und/oder Bewegung der aktuierbaren Komponente relativ zu der stationären Komponente in sechs linear unabhängigen Freiheitsgraden steuerbar ist.

Die genaue Zahl der hierzu erforderlichen Spuleneinheiten sowie deren Verschaltung, Anordnung und Form sind für den einzelnen Anwendungsfall festzulegen. Die Anmeldung stellt entsprechende Werkzeuge, die diese Festlegung ermöglichen, sowohl in allgemeinen Prinzipien als auch anhand konkreter Beispiele bereit.

Die Eigenschaft, dass durch Ansteuern einer beliebigen Kombination von höchstens sechs der Mehrzahl von Spuleneinheiten eine Position und/oder Bewegung der aktuierbaren Komponente relativ zu der stationären Komponente in sechs linear unabhängigen Freiheitsgraden steuerbar ist, wird hier auch als Auswahleigenschaft bezeichnet.

Durch das Vorliegen dieser Auswahleigenschaft kann die aktuierbare Komponente auf effiziente und kompakte Weise bezüglich der stationären Komponente in allen Freiheitsgraden sowohl gelagert als auch bewegt (etwa in Rotation versetzt und/oder oder translatorisch verschoben und/oder gekippt) werden. Der Effizienzvorteil ist dadurch begründet, dass mehrere, insbesondere alle ausgewählten, Spuleneinheiten zur Aktuierung in beliebigen Richtungen verwendet werden. Durch die Aufteilung auf mehrere Aktoren sinkt Beanspruchung jedes einzelnen Aktors. Da die Aktorverlustleistung quadratisch mit der Aktorbeanspruchung steigt, sinkt insgesamt die Summe der Verlustleistungen.

Auch ist durch diese Vorgabe eine Redundanz und somit Sicherheit im Betrieb (insbesondere Erstfehlersicherheit, bei Bedarf auch etwa Zweitfehlersicherheit) auf einfache Weise zu erzielen. Durch diese Sicherheit und die vergleichsweise einfache Konstruktion, die ermöglich wird, ist auch eine lange Standzeit erzielbar.

Die Eigenschaft, dass jede einzelne der Mehrzahl von Spuleneinheiten zum Erzeugen des jeweiligen variablen Magnetfelds derart eingerichtet ist, dass durch Wechselwirkung des jeweiligen variablen Magnetfelds mit der Magnetanordnung Kräfte und/oder Momente bezüglich mehrerer Freiheitsgrade auf die aktuierbare Komponente wirken, also dass jede (insbesondere unabhängige) Spuleneinheit die aktuierbare Komponente in mehreren Freiheitsgraden zu aktuieren vermag, wird hier als Universalaktoreigenschaft bezeichnet. Diese Eigenschaft macht die Aktoren untereinander austauschbar. Dies begünstigt insbesondere auch die Auswahleigenschaft.

Eine gegebene Spuleneinheit kann eine oder mehrere Spulen bzw. Windungen umfassen. Eine Spuleneinheit umfasst dabei insbesondere die Spulen bzw. Windungen, welche durch ihre Verschaltung nur gemeinsam, aber unabhängig von anderen Spuleneinheiten bestromt werden können.

Eine Spuleneinheit kann im Falle eines Elektromotors insbesondere einer Motorphase entsprechen. Ein Fehler, gegenüber dem durch die anmeldungsgemäße Baugruppe Sicherheit erzeugt wird, kann beispielsweise ein Bruch einer Spule oder ein Kurzschluss (eine nicht vorgesehene elektrische Verbindung) zwischen zwei Spulen oder eine Kombination solcher Fehler sein oder diese umfassen.

Die Eigenschaft, dass durch Ansteuern einer beliebigen Kombination von höchstens sechs der Mehrzahl von Spuleneinheiten eine Position und/oder Bewegung der aktuierbaren Komponente relativ zu der stationären Komponente in sechs linear unabhängigen Freiheitsgraden steuerbar ist, kann auch den Fall umfassen, dass die Steuerbarkeit in sechs linear unabhängigen Freiheitsgraden bereits durch eine beliebige Kombination einer vorgegebenen Zahl von Spuleneinheiten gegeben ist, wobei die vorgegebene Zahl weniger als sechs, beispielsweise fünf oder vier, beträgt. Für eine quasistatische Steuerbarkeit (siehe hierzu weiter unten) wird jedoch im Allgemeinen eine Auswahleigenschaft bezüglich sechs beliebiger Spulen erforderlich sein. Dies ist darin begründet, dass der Informationsgehalt eines Vorgabevektors, beispielsweise eines Spuleneinheitsstromvektors, mit weniger als sechs Einträgen nicht ausreicht, um sechs linear unabhängige Aktorkräfte oder Aktormomente eindeutig abzubilden.

Es kann vorgesehen sein, dass zu jeder vorgegebenen Spuleneinheit der Mehrzahl von Spuleneinheiten eine erste Position der aktuierbaren Komponente und eine zweite Position der aktuierbaren Komponente existieren,
so dass in der ersten Position durch Wechselwirkung der Magnetanordnung mit dem mittels der vorgegebenen Spuleneinheit erzeugbaren variablen Magnetfeld eine Kraft und/oder ein Moment erzeugbar ist, das bezüglich eines ersten Freiheitsgrades der sechs linear unabhängigen Freiheitsgrade auf die aktuierbare Komponente wirkt und
so dass in der zweiten Position durch Wechselwirkung der Magnetanordnung mit dem mittels der vorgegebenen Spuleneinheit erzeugbaren variablen Magnetfeld eine Kraft und/oder ein Moment erzeugbar ist, das bezüglich eines zweiten Freiheitsgrades der sechs linear unabhängigen Freiheitsgrade auf die aktuierbare Komponente wirkt.

Auf diese Weise trägt jede der Spuleneinheiten zur Aktuierbarkeit der aktuierbaren Komponente in mindestens zwei, oder auch mehreren Freiheitsgraden bei (über alle möglichen relativen Positionen der stationären Komponente und der aktuierbaren Komponente), was wiederum zur Kompaktheit, Effizienz und/oder Sicherheit der Anordnung beitragen kann. Diese Eigenschaft eignet sich als Kriterium, um die einzelnen Aktoren, bestehend aus Spuleneinheit und Magnetanordnung, isoliert zu konstruieren, auszulegen und zu optimieren. Die genaue Zahl der hierzu erforderlichen Spuleneinheiten sowie deren Verschaltung, Anordnung und Form sind für den einzelnen Anwendungsfall festzulegen. Die Anmeldung stellt entsprechende Werkzeuge, die diese Festlegung ermöglichen, sowohl in allgemeinen Prinzipien als auch anhand konkreter Beispiele bereit.

Es kann vorgesehen sein, dass zu jedem vorgegebenen der sechs Freiheitsgrade mindestens eine erste Spuleneinheit der Mehrzahl von Spuleneinheiten und eine zweite Spuleneinheit der Mehrzahl von Spuleneinheiten existieren,
so dass durch Wechselwirkung der Magnetanordnung mit dem mittels jeder einzelnen der ersten Spuleneinheit und der zweiten Spuleneinheit erzeugbaren variablen Magnetfeld eine Kraft und/oder ein Moment erzeugbar ist, das bezüglich des vorgegebenen Freiheitsgrades auf die aktuierbare Komponente wirkt.

Auf diese Weise tragen zur Aktuierbarkeit der aktuierbaren Komponente in jedem Freiheitsgrad mehrere Spuleneinheiten bei, was wiederum zur Kompaktheit, Effizienz und/oder Sicherheit der Anordnung beitragen kann. Insbesondere ist somit eine Redundanz für jeden Freiheitsgrad, und damit des gesamten Systems, gegeben. Während diese Konstruktionsvorschrift auch von Aktoren mit einzelner Redundanz erfüllt werden kann, so ist insbesondere in Verbindung mit der zuvor genannten Konstruktionsvorschrift, dass jeder Aktor mehrere Freiheitsgrade zu bedienen vermag, eine Gesamtkonstruktionsvorschrift für Einzelaktoren gegeben, aus welchen sich besonders kompakte und effiziente Lagerungssysteme zusammenstellen lassen. Die genaue Zahl der hierzu erforderlichen Spuleneinheiten sowie deren Verschaltung, Anordnung und Form sind für den einzelnen Anwendungsfall festzulegen. Die Anmeldung stellt entsprechende Werkzeuge, die diese Festlegung ermöglichen, sowohl in allgemeinen Prinzipien als auch anhand konkreter Beispiele bereit.

Jede einzelne der Mehrzahl von Spuleneinheiten kann so verschaltet sein, dass sie unabhängig von jeder anderen der Mehrzahl von Spuleneinheiten zum Erzeugen des jeweiligen variablen Magnetfelds ansteuerbar ist.

Eine solche Verschaltung kann insbesondere auf einfache Art zu den vorgenannten Eigenschaften hinsichtlich der Ansteuerbarkeit von Freiheitsgraden mittels der Spuleneinheiten beitragen oder diese Eigenschaften sicherstellen, ggf. zusammen mit einer geeigneten Wahl der Anzahl, Anordnung und/oder Form der Spuleneinheiten wie in dieser Anmeldung beschrieben. Unabhängig ansteuerbar bedeutet in diesem Kontext, dass beliebige Kombinationen von Phasenströmen einprägbar sind. Dafür darf auch eine Anpassung der Ansteuerung anderer Phasen notwendig sein. Ist ein Phasenstrom allerdings durch andere Phasenströme vorgegeben, dann ist dieser nicht unabhängig. Bei der üblichen dreiphasigen Sternschaltung mit aktiver Ansteuerung der drei Phasen und ohne aktive Ansteuerung des Sternpunkts, existieren nur zwei unabhängige Phasenströme, da der dritte Phasenstrom sich unmittelbar aus dem Kirchhoff'schen Gesetz ergibt. Damit handelt es sich zwar um ein dreiphasiges System, allerdings existieren nur zwei unabhängige Phasen beziehungsweise Spuleneinheiten im Sinne dieser Anmeldung. Grundsätzlich ist die Dimension der quasistationären Steuerbarkeit nach oben durch die Anzahl der unabhängigen Spuleneinheiten, beziehungsweise unabhängigen Phasen, begrenzt.

Die stationäre Komponente und die aktuierbare Komponente können einen Elektromotor, insbesondere einen Axialflussmotor, oder eine Komponente, insbesondere eine Axialfluss-Komponente, eines Elektromotors bilden. Dabei kann jede der Mehrzahl von Spuleneinheiten einer Motorphase des Elektromotors bzw. der Axialfluss-Komponente entsprechen.

Mit einer Axialfluss-Komponente ist hier insbesondere der Fall gemeint, dass ein Elektromotor sowohl Radialfluss- als auch Axialflussanteile umfasst (beispielsweise als Radial-Axial-Motor, Raxial-Motor oder Radax-Motor bezeichnet); in diesem Fall bezieht sich Axialfluss-Komponente auf die Axialflussanteile. Eine Axialfluss-Komponente kann in einem weiteren Beispiel auch durch die Vorspannung bei einer Kombination von zwei vorgespannten Radialflussmotoren gebildet werden.

Warum insbesondere die Axialflussanteile eine Steuerung in sechs Freiheitsgraden zulassen, wird deutlich, wenn die Steuerbarkeit eines konventionellen Radialflussmotors analysiert wird.

Die auf den mittig ausgerichteten Rotor eines Radialflussmotors übertragenen Kräfte greifen für alle Spulen des Radialflussmotors stets in radialer (durch die Rotationsachse verlaufend) oder tangentialer (die Rotationsachse nicht schneidend) Richtung am Rotor an, aber nie in axialer Richtung. Damit liegen alle erzeugbaren Kraftvektoren in einer Ebene. Selbst aus beliebig vielen Kraftvektoren einer Ebene lassen sich in Summe nur Nettokräfte in dieser Ebene, also zweidimensional, sowie Drehmoment senkrecht zu der Ebene, also eindimensional, generieren. Damit ist die Steuerbarkeit eines solchen reinen Radialflussmotors auf drei Dimensionen begrenzt.

Das Verfahren und bevorzugte Ausführungsformen werden im weiteren Verlauf anhand von Axialflussmotoren erläutert. Diese umfassen allerdings sämtliche Motoren mit Axialfluss-Anteilen, wie beispielsweise axial vorgespannte Radialflussmotoren.

Die Mehrzahl von Spuleneinheiten kann eine erste Gruppe von Spuleneinheiten und eine zweite Gruppe von Spuleneinheiten umfassen, wobei die Spuleneinheiten der ersten Gruppe zum Bilden eines ersten Axialflussmotor-Stators in einer ersten Ebene angeordnet sind und die Spuleneinheiten der zweiten Gruppe zum Bilden eines zweiten Axialflussmotor-Stators in einer zweiten Ebene angeordnet sind. Die Gruppen von Spuleneinheiten wirken bevorzugt auf dieselbe aktuierbare Komponente. Diese kann separate Magnetanordnungen für jede der Gruppen von Spuleneinheiten umfassen. Die Gruppen von Spuleneinheiten können jedoch auch auf zwei Seiten derselben Magnetanordnung wirken, um die aktuierbare Komponente leicht und kompakt zu halten und das magnetische Material besonders effizient zu nutzen. Solche Anordnungen sind für dreiphasige Axialflussmotoren bekannt, können in den bekannten symmetrischen Konfigurationen aber nur Drehmomente und keine Axialkräfte erzeugen und sind damit nur eindimensional steuerbar. Dabei müssen die einzelnen Gruppen von Spuleneinheiten nicht für sich alleine in sechs Freiheitsgraden steuerbar sein, stattdessen kann die volle Steuerbarkeit auch erst in Kombination erreicht werden.

In vorteilhaften Beispielen weisen der erste Axialflussmotoren-Stator und der zweite Axialflussmotor-Stator jeweils genau drei Spuleneinheiten auf und die Magnetanordnung weist ein, drei oder fünf Polpaare auf.

Es kann vorgesehen sein, dass die elektrische Baugruppe gekennzeichnet ist durch einen konstruktiv bedingten Versatz zwischen jeweiligen Wirkrichtungen und/oder Spulenachsen der Spuleneinheiten des ersten Axialflussmotor-Stators und des zweiten Axialflussmotor-Stators. Im Betrieb kann sich durch den konstruktiv bedingten Versatz ein Phasenversatz der Ansteuerung ergeben. Dies kann, wie weiter unten dargestellt, Vorteile für die Steuerbarkeit haben. Als Wirkrichtung wird hierbei eine Richtung bezeichnet, in der die jeweilige Spuleneinheit zum Erzeugen einer Kraft und/oder eines Moments auf die Magnetanordnung eingerichtet ist.

Die Wirkrichtung kann sich dabei auf die Wirkung einer auf die aktuierbare Komponente wirkenden Kraft an einem definierten Angriffspunkt mit einer definierten Richtung beziehen. Zusätzlich oder alternativ kann sich die Wirkrichtung auf Drehmomente um definierte Drehachsen beziehen. Der Versatz kann bezüglich der Angriffspunkte, der Richtungen der Kräfte oder der Achsen der Drehmomente gegeben sein, ist jedoch nicht auf diese beschränkt. Allgemein soll der Versatz verhindern, dass der Beitrag der Spuleneinheit oder Phase zur Aktuierung, dargestellt als Kraft-Moment-Vektor, linear abhängig ist zum Beitrag der anderen Phasen oder Spuleneinheiten, und somit die Steuerbarkeit degradiert.

Der konstruktiv bedingte Versatz ist insbesondere durch einen rotatorischen Versatz einer Anordnung der Spuleneinheiten des ersten Axialflussmotor-Stators gegenüber einer Anordnung der Spuleneinheiten des zweiten Axialflussmotor-Stators und/oder durch einen rotatorischen Versatz jeweiliger Polpaare zweier Magnetanordnungen gegeneinander und/oder durch eine schiefwinklige Magnetisierung einer gemeinsamen Magnetanordnung erzielbar. Es kann vorgesehen sein, dass durch Ansteuern einer beliebigen Kombination von höchstens fünf, insbesondere höchstens vier oder höchstens drei, der Mehrzahl von Spuleneinheiten eine Position und/oder Bewegung der aktuierbaren Komponente relativ zu der stationären Komponente unter Ausnutzung einer Trägheit der aktuierbaren Komponente in einem bewegten, insbesondere rotierenden, Zustand der aktuierbaren Komponente in den sechs linear unabhängigen Freiheitsgraden steuerbar ist.

Eine Steuerbarkeit dieser Art, die also die Trägheit der aktuierbaren Komponente im bewegten Zustand ausnutzt, um insgesamt alle betreffenden Freiheitsgrade ansteuern zu können, wird hier auch als dynamische Steuerbarkeit bezeichnet. Davon abzugrenzen ist eine quasistatische Steuerbarkeit, bei der alle betreffenden Freiheitsgrade auch dann angesteuert werden können, wenn die aktuierbare Komponente bezüglich der stationären Komponente in Ruhe ist.

Eine quasistatische Steuerbarkeit in allen sechs linear unabhängigen Freiheitsgraden kann insbesondere auf Grundlage der oben genannten Auswahleigenschaft bezüglich sechs beliebiger Spuleneinheiten gegeben sein.

In einem Beispiel kann eine quasistatische Steuerbarkeit auf Grundlage der Auswahleigenschaft bezüglich sechs beliebiger Spuleneinheiten mit einer dynamischen Steuerbarkeit mittels einer geringeren Anzahl von Spuleneinheiten wie oben genannt (also dergestalt, dass durch Ansteuern einer beliebigen Kombination von höchstens fünf, insbesondere höchstens vier oder höchstens drei, der Mehrzahl von Spuleneinheiten eine Position und/oder Bewegung der aktuierbaren Komponente relativ zu der stationären Komponente unter Ausnutzung einer Trägheit der aktuierbaren Komponente in einem bewegten, insbesondere rotierenden, Zustand der aktuierbaren Komponente in den sechs linear unabhängigen Freiheitsgraden steuerbar ist) kombiniert werden. Diese Kombination kann in einem Steuerverfahren ausgenutzt werden, bei dem von einer quasistatischen Ansteuerung im Normalbetrieb zu einer dynamischen Ansteuerung in einem Fehlerzustand, der die Funktionsfähigkeit mindestens einer Spuleneinheit einschränkt, gewechselt werden kann, wie weiter unten beschrieben.

Es kann vorgesehen sein, dass die Mehrzahl von Spuleneinheiten höchstens neun oder höchstens acht, insbesondere genau neun, genau acht, genau sieben oder genau sechs, Spuleneinheiten umfasst.

Die obige Auswahleigenschaft lässt sich durch eine solche Wahl der Anzahl von Spuleneinheiten auf besonders kompakte, wirtschaftliche und/oder effiziente Weise bereitstellen (im Falle von sechs Spulen auf triviale Weise, da hier nur eine Auswahl von sechs Spuleneinheiten möglich ist). Eine detaillierte Analyse hinsichtlich der Steuerbarkeit im Falle verschiedener Konfigurationen hinsichtlich der Zahl und Anordnung mehrerer - insbesondere unabhängig verschalteter - Spuleneinheiten wird weiter unten gegeben.

In einem vorteilhaften Beispiel umfasst die Mehrzahl von Spuleneinheiten genau sieben Spuleneinheiten und die Magnetanordnung der aktuierbaren Komponente umfasst genau zwei, fünf oder neun Polpaare. In einem weiteren vorteilhaften Beispiel umfasst die Mehrzahl von Spuleneinheiten genau acht Spuleneinheiten und die Magnetanordnung der aktuierbaren Komponente umfasst genau zwei oder sechs Polpaare, wobei ein Vorteil in der Steuerbarkeit in sechs Freiheitsgraden liegt.

Bezüglich der Magnetanordnung der aktuierbaren Komponente kann vorgesehen sein, dass diese zwischen einem und neun magnetischen Polpaaren umfasst, insbesondere zwei, fünf, sechs oder neun Polpaare. Wie in dieser Anmeldung gezeigt wird, ist beispielsweise eine Zahl von zwei, fünf oder neun Polpaaren besonders geeignet in Kombination mit einer stationären Komponente mit sieben Spuleneinheiten, eine Zahl von zwei oder sechs Polpaaren besonders geeignet in Kombination mit einer stationären Komponente mit acht Spuleneinheiten usw., um eine Steuerbarkeit in sechs Freiheitsgraden zu erreichen (siehe Steuerbarkeitsanalyse weiter unten).

Damit, dass die Mehrzahl von Spuleneinheiten eine vorgegebene Höchstzahl oder eine vorgegebene genaue Anzahl von Spuleneinheiten umfasst (hier also höchstens acht, genau sieben oder genau sechs Spuleneinheiten), ist gemeint, dass die stationäre Komponente neben dieser Höchstzahl oder genauen Anzahl von Spuleneinheiten, die die Auswahleigenschaft erfüllen, keine weiteren Spuleneinheiten umfasst, die ebenfalls (zusammen mit den vorgenannten Spuleneinheiten) die Auswahleigenschaft erfüllen. Dies kann etwa bedeuten, dass die stationäre Komponente überhaupt keine weiteren Spulen oder Spuleneinheiten umfasst, kann aber auch den Fall umfassen, dass die stationäre Komponente zusätzliche Spulen mit anderer Funktion (etwa Sensorspulen etc.) umfasst, die aber nicht zusammen mit den vorgenannten Spuleneinheiten die Auswahleigenschaft erfüllen.

Die elektrische Baugruppe kann eine Stützvorrichtung zum Stützen der aktuierbaren Komponente in einem Freiheitsgrad derart umfassen, dass die aktuierbare Komponente in diesem Freiheitsgrad relativ zu der stationären Komponente passiv stabil gelagert ist. Die Stützvorrichtung ist vorzugsweise dazu eingerichtet, eine erste und eine zweite Position einzunehmen, so dass die Stützvorrichtung mit der aktuierbaren Komponente in der ersten Position zum genannten Stützen in Eingriff ist und in der zweiten Position nicht in Eingriff ist. Mittels einer solchen Stützvorrichtung kann insbesondere ein Bestimmen von Querabhängigkeiten oder Verkopplungen zwischen einzelnen Freiheitsgraden ermöglicht werden, wie weiter unten beschrieben. Die Stützvorrichtung kann in der zweiten Position nicht mehr Teil der elektrischen Baugruppe, also entfernbar ausgelegt, sein.

Die stabilisierende Unterstützung durch die Stützvorrichtung kann hinreichend sein, um die aktuierbare Komponente so weit zu stabilisieren, dass diese passiv in allen Freiheitsgraden gelagert ist. In dieser Konfiguration kann eine Vermessung der Charakteristik der Positionssensoren und/oder der von den Spulen erzeugten Kräfte und Momente erfolgen. Mit dieser Vermessung können Parameter des Regelkreises, insbesondere die Einträge der Km-Matrix oder die Parameter der Freiheitsgradregler, bestimmt werden.

Die stationäre Komponente und/oder die aktuierbare Komponente kann bzw. können einen Beschleunigungssensor und/oder einen Kraftsensor umfassen. Damit kann beispielsweise ein Erfassen und/oder Ausgleichen von Unwuchten ermöglicht werden, wie weiter unten beschrieben.

Es kann vorgesehen sein, dass das mittels einer ersten der Mehrzahl von Spuleneinheiten erzeugbare Magnetfeld asymmetrisch, insbesondere hinsichtlich Rotation und/oder oder Spiegelung, in Bezug auf das mittels einer zweiten der Mehrzahl von Spuleneinheiten erzeugbare Magnetfeld ausgeführt ist. Es kann vorgesehen sein, das jeweilige magnetische Komponenten, etwa Spulen und/oder Statorzähne, mindestens zweier Spuleneinheiten der Mehrzahl von Spuleneinheiten zueinander asymmetrisch, insbesondere nicht äquidistant, angeordnet und/oder unterschiedlich dimensioniert und/oder unterschiedlich geformt sein. Alternativ oder zusätzlich können magnetische Komponenten der Magnetanordnung der aktuierbaren Komponente zueinander asymmetrisch, insbesondere nicht äquidistant, angeordnet und/oder unterschiedlich dimensioniert und/oder unterschiedlich geformt sein.

Durch die in diesem Sinne asymmetrische Felderzeugung und/oder asymmetrische/unregelmäßige Anordnung und/oder Ausgestaltung von Komponenten kann die Steuerbarkeit, insbesondere eine Ausfallsicherheit der Steuerbarkeit, der aktuierbaren Komponente verbessert werden, wie weiter unten noch näher erläutert wird.

Ein offenbartes Steuerverfahren eignet sich zum Ansteuern einer elektrischen Baugruppe, umfassend eine stationäre Komponente mit einer Mehrzahl von Spuleneinheiten, wobei jede der Mehrzahl von Spuleneinheiten zum Erzeugen eines variablen Magnetfelds ansteuerbar ist, und mit einer aktuierbaren Komponente, die relativ zu der stationären Komponente beweglich ist und eine Magnetanordnung umfasst. Insbesondere eignet sich das Steuerverfahren zum Ansteuern einer elektrischen Baugruppe der oben diskutierten Art, die die Auswahleigenschaft erfüllt, in ihren verschiedenen möglichen Ausgestaltungen.

Das Steuerverfahren umfasst:
Bestimmen einer Mehrzahl von Steuervorgaben zum Ansteuern jeder der Mehrzahl von Spuleneinheiten,
Steuern und/oder Regeln einer Position und/oder Bewegung der aktuierbaren Komponente relativ zu der stationären Komponente durch Beaufschlagen der Mehrzahl von Spuleneinheiten mit einem Signal gemäß der bestimmten Mehrzahl von Steuervorgaben.

Es kann vorgesehen sein, dass die Mehrzahl von Steuervorgaben als Steuervorgabenvektor unter Verwendung einer Matrixmultiplikation einer Kommutierungsmatrix, insbesondere einer von der Position der aktuierbaren Komponente abhängigen Kommutierungsmatrix, mit einem Kraft-Moment-Vektor bestimmt wird. Der Kraft-Moment-Vektor ist ein Vektor mit Kraft- und/oder Momenteinträgen, die sich insbesondere auf Kräfte bzw. Momente beziehen, die durch geeignetes Ansteuern der Mehrzahl von Spuleneinheiten auf die aktuierbare Komponente ausübbar sind.

Es kann vorgesehen sein, dass die Kommutierungsmatrix auf Grundlage einer Verknüpfungsmatrix unter Berücksichtigung mindestens eines Optimierungskriteriums, beispielsweise einer Minimierung einer Verlustleistung und/oder einer Maximierung einer Steuerbarkeit, bestimmt wird. Die Verknüpfungsmatrix ist dabei so definiert, dass sich der Kraft-Moment-Vektor (ggf. bis auf Rechen- oder Optimierungsfehler) durch Matrixmultiplikation aus der Verknüpfungsmatrix und dem Steuervorgabenvektor ergibt.

Es kann vorgesehen sein, dass die Kommutierungsmatrix unter Verwendung einer Simulation, beispielsweise mittels der Finite-Elemente-Methode, und/oder unter Verwendung einer Messung bestimmt wird. Eine solche Messung kann beispielsweise mittels Kraft- und/oder Momentsensoren an der aktuierbaren Komponente erfolgen. Eine Messung kann auch durch Bewegung der aktuierbaren Komponente in verschiedenen Richtungen, insbesondere den linear unabhängigen Freiheitsgraden, und Erfassen einer durch diese Bewegungen in den Spuleneinheiten induzierten elektromotorischen Gegenkraft (Back-EMF) erfolgen.

Es kann vorgesehen sein, dass die Kommutierungsmatrix als mindestens dreidimensionale Steuervorschrift für einen mindestens vierphasigen Motor darstellbar ist. Die Implementation einer Steuervorschrift als Matrixmultiplikation ist für ein- oder zweidimensionale Steuerungen im Rahmen der feldorientierten Regelung bekannt, bei der die zwei Dimensionen Drehmoment und Feldschwächung umfassen. Ein System kann auch mehrere dieser zweidimensionalen feldorientierten Regelungen aufweisen. Der Vorteil der drei- bis sechsdimensionalen Steuervorschrift gegenüber mehreren zweidimensionalen Steuervorschriften ist, dass zur Korrektur von Verkopplungen oder zur Anwendung des Auswahlkriteriums bei Ausfall einer Phase die elektrische Baugruppe einfach korrigiert werden kann, da das Systemverhalten mit einer Matrix beschrieben wird und somit mit geringem Aufwand global optimiert werden kann.

Das Steuerverfahren kann umfassen: Bestimmen einer Querabhängigkeit, also einer Abhängigkeit des Steuerns und/oder Regelns der Position und/oder Bewegung der aktuierbaren Komponente relativ zu der stationären Komponente bezüglich eines ersten Freiheitsgrades von der Position und/oder Bewegung der aktuierbaren Komponente relativ zu der stationären Komponente bezüglich eines zweiten Freiheitsgrades.

Das Steuerverfahren kann umfassen: Bestimmen und/oder Anpassen der Steuervorgaben unter Berücksichtigung der bestimmten Querabhängigkeit, insbesondere zum Minimieren der Querabhängigkeit.

Das Steuerverfahren kann umfassen: Minimieren einer Unwucht der aktuierbaren Komponente bezüglich einer Rotation relativ zu der stationären Komponente um eine Rotationsachse durch dynamisches Festlegen und/oder Anpassen einer Position und/oder Orientierung der Rotationsachse in Bezug auf die aktuierbare Komponente, insbesondere basierend auf mittels eines an der aktuierbaren Komponente und/oder stationären Komponente angeordneten Beschleunigungssensors und/oder Kraftsensors erfassten Sensordaten und/oder basierend auf einer Minimierung mindestens eines der Einträge des Kraft-Moment-Vektors.

Ein solches Minimieren einer Unwucht (auch als Auswuchten bezeichnet), ggf. einschließlich des entsprechenden Erfassens von Sensordaten, kann über unterschiedliche Zeiträume erfolgen, beispielsweise über mehrere oder viele Rotationen der aktuierbaren Komponente bezüglich der stationären Komponente, aber auch über kürzere Zeiträume, beispielsweise eine Rotation oder weniger. Die genauen Methoden sind aus fünfdimensionalen Magnetlagerungen mit zumeist individuellen Magnetlagern für jeden Freiheitsgrad bekannt. Die Berechnung der korrigierten Rotationsachse oder von Kompensationskräften anhand von Aktorkräften oder Positions- bzw. Beschleunigungssensordaten sind auch hier anwendbar. Allgemein sind Verfahren zum aktiven Auswuchten mehrdimensionaler Magnetlager aus dem Stand der Technik bekannt (z. Bsp. US9479035B2). Diese Methoden sind ebenso bei der vorliegenden elektrischen Baugruppe anwendbar. Hierzu kann die Sensorposition wie auch die Aktorkräfte mit den unten beschriebenen Verfahren in die einzelnen Freiheitsgrade zerlegt werden und anschließend wie separate Aktoren ausgewuchtet werden. Damit können auch sämtliche nur ein- oder zweidimensionale Auswuchtverfahren oder Störkraftkompensationsverfahren (z. Bsp. EP4249039A1) jeweils einzeln für die Freiheitsgrade angewendet werden.

Neben Unwuchten ist auch der Betrieb bei hohen Drehzahlen durch die sich schnell ändernden Steuervorgaben und Phasenströme eine Herausforderung. Folgend ist daher eine Weiterbildung des Steuerverfahrens dargelegt, welche die Lageregelung auch bei hohen Drehzahlen ermöglicht.

Das Bestimmen der Mehrzahl von Steuervorgaben kann umfassen: Bestimmen eines jeweiligen Phasenstroms durch jede der Mehrzahl von Spuleneinheiten.

Das Bestimmen der Mehrzahl von Steuervorgaben kann dann ferner umfassen: Transformieren der bestimmten Phasenströme in Anteile eines Freiheitsgrad-Stromvektors, die einer jeweiligen Verschiebung der aktuierbaren Komponente in Richtung der Achsen eines bezüglich der stationären Komponente ortsfesten Koordinatensystems und/oder einer jeweiligen Drehung der aktuierbaren Komponente um die Achsen dieses ortsfesten Koordinatensystems entsprechen.

Das Bestimmen der Mehrzahl von Steuervorgaben kann weiterhin umfassen: Bestimmen einer jeweiligen transformierten Steuervorgabe, insbesondere einer Stromvorgabe, für jede der Koordinaten des ortsfesten Koordinatensystems unter Verwendung eines jeweiligen Reglers.

Die Anteile des Freiheitsgrad-Stromvektors können anschließend mit den entsprechenden Anteilen der Stromvorgabe verglichen werden und somit die Anteile eines Freiheitsgrad-Stromfehlervektor gebildet werden.

Anhand der einzelnen Stromfehleranteile können die Ströme, welche zu einer Aktuierung in den gewählten Freiheitsgraden führen, einzeln und unabhängig von den anderen Freiheitsgraden geregelt werden. Die geregelten Ströme existieren in der Regel nicht eins-zu-eins in den Statorphasen, sondern stellen vorzugsweise sinnvolle Kombinationen von Phasenströmen dar, welche von dem Magnetfluss der in der aktuierbaren Komponente enthaltenen Magnetanordnung relativ zur stationären Komponente abhängen. Diese Orientierung der zu regelnden Ströme am tatsächlichen Magnetfluss ermöglicht es die individuellen Stromregler von den Rotorbewegungen, insbesondere einer schnellen Rotation, zu entkoppeln.

Das Bestimmen der Mehrzahl von Steuervorgaben kann umfassen: Rücktransformieren der transformierten Steuervorgaben in ein statorfestes (d. h. bezüglich der stationären Komponente ortsfestes) Bezugssystem zum Bestimmen der Mehrzahl von Steuervorgaben, insbesondere Phasenspannungen oder Phasenströme.

Eine Regelung mit Transformieren und Rücktransformieren wie angegeben kann als mehrdimensionale Variante einer feldorientierten Regelung aufgefasst werden und eignet sich beispielsweise für schnell rotierende Motoren, bei denen - bedingt durch schnell veränderliche Regelvorgaben etwa in Reglerendstufen - eine Phasenverschiebung zwischen Soll- und Ist-Strömen auftreten kann.

Das Steuerverfahren kann umfassen: Erfassen mindestens einer Messgröße, die ein Erfassen eines Fehlerzustands der elektrischen Baugruppe ermöglicht, wobei der Fehlerzustand insbesondere ein Ausfallen mindestens einer ersten Spuleneinheit der Mehrzahl von Spuleneinheiten und/oder eine nicht vorgesehene elektrische Verbindung mindestens zweier Spuleneinheiten der Mehrzahl von Spuleneinheiten umfasst.

Ein Erfassen eines solchen Fehlerzustands ermöglicht beispielsweise ein Ausgeben einer Warnung und/oder ein Beenden eines Betriebs der elektrischen Baugruppe, aber auch ein entsprechendes Anpassen des Betriebs, um trotz des Fehlerzustands den Betrieb aufrechterhalten zu können. So kann jeweils die Zuverlässigkeit und/oder Sicherheit im Betrieb verbessert werden.

Bei einem Erfassen des Fehlerzustands kann also insbesondere vorgesehen sein: Anpassen einer Vorschrift zum Bestimmen der Mehrzahl von Steuervorgaben derart, dass das Steuern und/oder Regeln der Position und/oder Bewegung der aktuierbaren Komponente relativ zu der stationären Komponente unter Verwendung der Mehrzahl von Spuleneinheiten unter Berücksichtigung des Fehlerzustands, insbesondere ohne die erste Spuleneinheit, erfolgt.

Es kann beispielsweise vorgesehen sein, dass nach Anpassen der Vorschrift durch Ansteuern einer beliebigen Kombination von höchstens fünf der Mehrzahl von Spuleneinheiten eine Position und/oder Bewegung der aktuierbaren Komponente relativ zu der stationären Komponente unter Ausnutzung einer Trägheit der aktuierbaren Komponente in einem bewegten, insbesondere rotierenden, Zustand der aktuierbaren Komponente in den sechs linear unabhängigen Freiheitsgraden steuerbar ist.

Es kann also insbesondere von einer quasistatischen Ansteuerung im Normalbetrieb zu einer dynamischen Ansteuerung bei Erfassen des Fehlerzustands gewechselt werden.

Eine offenbarte Steuereinheit ist eingerichtet zum Ansteuern einer elektrischen Baugruppe, insbesondere einer Baugruppe der oben beschriebenen Art in ihren verschiedenen Ausgestaltungen. Das Ansteuern erfolgt vorzugsweise gemäß einer oder mehrerer Ausgestaltungen des vorbeschriebenen Steuerverfahrens.

Ein offenbartes elektrisches System umfasst eine elektrische Baugruppe, insbesondere eine Baugruppe der oben beschriebenen Art in ihren verschiedenen Ausgestaltungen, sowie eine Steuerschnittstelle, mittels derer die Baugruppe, insbesondere die stationäre Komponente der Baugruppe, mit einer Steuereinheit, insbesondere der oben genannten Steuereinheit, verbindbar oder verbunden ist.

Die elektrische Baugruppe kann - wie in obigen Beispielen bereits erwähnt - eine Rotationsmaschine oder einen Teil einer Rotationsmaschine bilden, insbesondere einen Elektromotor oder einen Teil eines Elektromotors, wobei die stationäre Komponente ein Stator des Elektromotors ist und wobei die bewegliche Komponente ein Rotor des Elektromotors ist. Das elektrische System ist in diesem Fall insbesondere ein Motor- bzw. Rotationsmaschinensystem.

Der Rotor kann relativ zu dem Stator in mindestens einem Freiheitsgrad, insbesondere in allen sechs linear unabhängigen Freiheitsgraden, magnetisch gelagert oder lagerbar, insbesondere aktiv magnetisch gelagert oder lagerbar, sein. Der Rotor kann auch mechanisch und/oder hydrodynamisch gelagert oder lagerbar sein. Es kann eine Kombination von mehreren Lagerarten (mechanisch, hydrodynamisch, aktiv magnetisch, passiv magnetisch usw.) für verschiedene Freiheitsgrade (oder auch zusammenwirkend für einen Freiheitsgrad) vorgesehen sein.

Das elektrische System kann als Pumpsystem für ein gasförmiges Fördermedium und/oder flüssiges Fördermedium ausgebildet sein. Neben Förderpumpen, die ein Fördermedium von einem ersten Ort zu einem zweiten Ort befördern, wie etwa Kraftstoffpumpen oder Vakuumpumpen, umfasst dies beispielsweise auch Umwälzpumpen, die das Fördermedium innerhalb eines Reservoirs durchmischen.

Beispielsweise kommen magnetisch gelagerte Pumpen dort zum Einsatz, wo reibende Dichtungen, Schmierstoffe, oder Kugellager nachteilig sind, etwa wegen Anforderungen an Kontamination, Reibleistung, Vibration oder chemische Kompatibilität. Allerdings sind magnetisch gelagerte Pumpen empfindlicher gegenüber Bewegungen, da große aktive Kräfte notwendig sind, um den Rotor der Bewegung des Stators nachzuführen. Um diesen Nachteil zu reduzieren, kann der Rotor auftriebsausgeglichen ausgeführt sein. Das heißt, dass die mittlere Dichte des Rotors durch Hohlräume oder Zusatzmassen der Dichte des Fördermediums angepasst wird. Fällt der Schwerpunkt des Rotors mit dem Auftriebszentrum des verdrängten Fluids zusammen, dann sind zusätzlich auch Beschleunigungen und Drehbewegungen ausgeglichen. Nicht ausgeglichene gyroskopische Momente können jedoch unter Umständen weiterhin auftreten und können aktiv ausgeglichen werden.

Ein Auftriebsausgleich der aktuierbaren Komponente kann auch für besonders vibrationsarme Antriebe vorteilhaft sein. Der Antriebsausgleich reduziert die zur Levitation notwendigen statischen Kräfte. Sind diese Kräfte ausreichend klein, dann kann auf passive magnetische Komponenten wie Rückflusseisen oder passive Magnetlager verzichtet werden. Damit sind die von den Spuleneinheiten generierten Kräfte die einzigen magnetischen Kräfte, die auf den Rotor wirken. Da diese Kräfte zum einen genau bekannt sind und zum anderen genau eingestellt werden können, wird ein sehr genaues aktives Auswuchten ermöglicht. Hierfür kann der Auftriebsausgleich in Kombination mit weiteren die Rotorkraft reduzierenden Maßnahmen eingesetzt werden, wie beispielsweise Trag- und Deckscheiben, Doppelvoluten oder einem zweiseitigen axialen Einlass für Radialpumpen.

Das elektrische System kann als Herzunterstützungssystem und/oder Fontanzirkulationsunterstützungssystem ausgebildet sein, wobei die stationäre Komponente ein Stator einer Rotationsfluidpumpe zum Fördern von Blut (Rotationsblutpumpe oder kurz Blutpumpe) ist, wobei die aktuierbare Komponente ein Rotor der Rotationsfluidpumpe ist. Ausfallsicherheit (insbesondere Erstfehlersicherheit), Kompaktheit und Effizienz sind bei Blutpumpen von besonderer Bedeutung. Eine Steuerbarkeit (insbesondere in Gestalt einer Regelung) in allen sechs Freiheitsgraden hat zudem in diesem Zusammenhang besondere Vorteile im Hinblick auf die Laufruhe einer Blutpumpe (sowohl aufgrund der guten Dämpfung durch aktiv magnetische Lagerbarkeit des Rotors in allen Freiheitsgraden als auch durch die Möglichkeit der Kompensation von Unwuchten wie beschrieben).

Die elektrische Baugruppe kann als Reaktionsrad, insbesondere zur Lageregelung eines Flugkörpers, ausgebildet sein. Der Flugkörper kann beispielsweise ein Satellit, ein Luft- oder Raumfahrzeug (einschließlich Rakete, Flugzeug, Drohne) sein. Ein Reaktionsrad umfasst eine rotierbare Schwungmasse, die insbesondere mit der aktuierbaren Komponente (als Rotor ausgeführt) gekoppelt und/oder durch diese gebildet sein kann. Im Betrieb wird der Rotor mit Schwungmasse beschleunigt oder abgebremst, um durch Drehmomenterhaltung den Satelliten in der Rotation zu beschleunigen oder abzubremsen. Die Lagerung des Rotors des Reaktionsrads erfolgt zumeist mit Kugellagern. Diese haben jedoch eine begrenzte Drehzahl beziehungsweise Lebensdauer und können durch elektrostatische Entladungen oder einen alternden Schmierstoff vorzeitig ausfallen. Keramik-basierte Kugellager adressieren diese Probleme bisher. Doch auch magnetlagerbasierte Reaktionsräder wurden entwickelt, allerdings mit separatem Motor und Magnetlagern für verschiedene Freiheitsgrade. Eine besondere Anwendung magnetisch gelagerter Reaktionsräder ist die Kompensation von Mikrovibrationen, welche durch den Einsatz von Kugellagern entstehen können oder von anderen Komponenten im Flugkörper ausgehen können. Insbesondere können nicht nur Rotationsschwingungen, sondern im Gegensatz zu kugelgelagerten Reaktionsrädern auch translatorische Schwingungen reduziert oder auch aktiv kompensiert werden.

Ein weiterer Vorteil von aktiv magnetisch gelagerten Reaktionsrädern ist, dass die Rotationsachse gegenüber der stationäre Komponente, in diesem Fall fixiert am Flugkörper, verschoben werden kann. Bei einer Lagerung mit Kugellagern ist die Rotationsachse nicht verschiebbar. Es kann daher nur der eine fixe Komponente des Drehimpulses mit dem kugelgelagerten Reaktionsrad beeinflusst werden. Für eine Ausrichtung des Flugkörpers in mehreren Achsen sind mehrere Reaktionsräder oder eine Aufhängung des Reaktionsrads in einem aktiven Gimbal, also mehrere Aktorelemente, erforderlich. Um die bestimmte Ausrichtung zu halten oder kleinere Ausrichtungskorrekturen durchzuführen ist es ausreichend, die Rotationsachse des nach Möglichkeit drehenden Reaktionsrads zu verkippen oder die Drehzahl des Reaktionsrad zu verändern. Für größere Kurskorrekturen kann das Reaktionsrad zunächst angehalten werden und anschließend um eine erste Achse beschleunigt und anschließend abgebremst werden. Der Flugkörper wird sich daraufhin um eine erste Flugkörperachse neu ausrichten. Anschließend kann die Rotationsachse des Reaktionsrades in einer zweiten Rotationsachse ausgerichtet werden und das Reaktionsrad in dieser Konfiguration beschleunigt und abgebremst werden. Der Flugkörper richtet sich daraufhin in einer zweiten Flugkörperachse aus. Die Position des Reaktionsrads bezüglich der stationären Komponente und der Gesamtdrehimpuls des Flugkörpers haben sich allerdings nicht verändert. Durch wiederholtes Verschieben der Rotationsachse sowie Beschleunigen und Abbremsen des Reaktionsrads in Rotationsrichtung kann der Flugkörper schrittweise beliebig ausgerichtet werden, obwohl die Rotationsachse des Reaktionsrades nur in einem begrenzten Bereich verstellt wurde. Hierdurch können mehrere Reaktionsräder eingespart werden oder mit ausgefallenen Reaktionsrädern ein Notbetrieb gewährleistet werden.

Die anmeldungsgemäße elektrische Baugruppe kann sowohl den Motor als auch alle aktiven Magnetlager ersetzen und bietet zudem eine Erstfehlersicherheit mit geringen Komplexitäts- oder Gewichts-Kosten.

Weitere Anwendungsmöglichkeiten umfassen beispielsweise Lüfter, Kompressoren, Mischer, Turbinen, Zentrifugen und andere Rotationsmaschinen.

Die in den meisten solchen Anwendungen (ebenso wie für Pumpen, siehe oben) eingesetzten Lager wie Kugellager, Gleitlager oder hydrodynamische Lager, haben für einige Spezialanwendungen inakzeptable Nachteile wie Unwucht, Verschleiß, Kontamination oder fehlende Trockenlauffähigkeit. Die Spezialanwendungen, welche deshalb auf Magnetlager setzen, lassen sich in zwei verschiedene Kategorien unterteilen:
i. vollständig aktive Magnetlagerung mit einer Phase für jeden Freiheitsgrad,
ii. Passive Magnetlagerung mit aktiver Magnetlagerung in einer Untermenge von Freiheitsgraden.
Wesentliche Nachteile einer konventionellen vollständig aktiven Magnetlagerung sind aufwändige Redundanzkonzepte und eine ineffiziente Ausnutzung des Bauraums.

Ein Nachteil der teilweise passiven Magnetlagerung ist, dass ein passives Magnetlager sich bezüglich des Zusammenhangs zwischen Rückstellkraft und Auslegung wie eine Feder mit positiver oder negativer Federkonstante verhält. In Verbindung mit der Rotormasse ergibt sich ein Feder-Masse-Schwinger, welcher, insbesondere bei der Resonanzfrequenz, aufzuschwingen vermag. Gedämpft wird dieser Schwinger durch das Fluid oder zusätzliche Dämpfungselemente wie Wirbelstromdämpfer. Insbesondere mit Gas als Fördermedium ist die Dämpfung sehr klein und die Resonanzüberhöhung kann sehr groß werden. Selbst wenn die Maschine selbst nicht in der Nähe der Resonanzfrequenz betrieben wird, dann können benachbarte Maschinen, oder in mobilen Anwendungen die Bewegung der Maschine, die Resonanzen dennoch anregen. Zudem bleiben Beschädigungen durch einen Kontakt zwischen Rotor und Gehäuse oft unerkannt, da die Rotorposition in dieser Achse häufig nicht gemessen wird, da dies nicht für die Lageregelung erforderlich ist.

Die Nachteile beider Ansätze lassen sich durch Verwendung der anmeldungsgemäßen elektrischen Baugruppe adressieren. Hierbei kann die Lagerung selber vollständig durch die, optional auch fehlersichere, Baugruppe erfolgen. Es kann jedoch auch sinnvoll sein, den Motor durch passive Magnetlager zu unterstützen. Hierbei werden einzelne Freiheitsgrade entweder stabil oder instabil magnetisch gelagert, wobei die instabilen Freiheitsgrade nach dem Earnshaw-Theorem in der Summe der Federsteifigkeiten überwiegen oder die Summe ausgeglichen ist. In allen magnetisch gelagerten Freiheitsgraden kann eine Null-Kraft-Regelung oder auch Zero-Power-Control realisiert werden, um die Lagerkräfte mit sehr wenig Verlustleistung aufzunehmen. Ebenso können alle Freiheitsgrade aktiv gedämpft werden und die Maschine so unempfindlich gegenüber Bewegungen oder Störung durch benachbarte Maschinen gestaltet werden.

Für Applikation bei denen eine geringe Systemkomplexität oder geringe Kosten im Vordergrund stehen kann ein Motor mit 6 oder sogar hinab bis zu 3 unabhängigen Phasen zum Einsatz kommen. Die Regelung und insbesondere Dämpfung erfolgt in diesem Fall mit der vorgestellten unterbestimmten Regelung.

Auch Systeme mit nicht-rotierenden elektrischen Baugruppen stellen vorteilhafte Anwendungsmöglichkeiten dar. Das elektrische System kann etwa als Stabilisationsvorrichtung, insbesondere für ein optisches Abbildungssystem, ausgebildet sein. Das optische Abbildungssystem kann beispielsweise ein Abbildungssystem einer Kamera, eines Teleskops, eines Belichtungssystems usw. sein. Es kann beispielsweise vorgesehen sein, durch geeignete Ansteuerung der aktuierbaren Komponente einen Bildsensor, einen Spiegel, eine abbildende Linse oder Linsenanordnung oder eine sonstige optische Komponente in ihrer Position und/oder Orientierung zu stabilisieren.

Die nachfolgend beschriebenen Zeichnungen illustrieren Hintergründe, Prinzipien und beispielhafte Ausführungsformen des Gegenstands der Anmeldung. Dabei zeigen, jeweils schematisch und nicht in einem vorgegebenen Maßstab,
FIG. 1A eine perspektivische Ansicht einer elektrischen Baugruppe,
FIG. 1B eine Draufsicht eines Teils der elektrischen Baugruppe nach FIG. 1A,
FIG. 1C einen Längsschnitt durch die Schnittebene A--A der elektrischen Baugruppe nach FIG. 1A,
FIG. 2 ein Diagramm, das die Anzahl steuerbarer Freiheitsgrade für verschiedene elektrische Baugruppen zeigt,
FIG. 3 einen Verlauf von Magnetfeldern in Relation zu Spulenpositionen bei einer beispielhaften elektrischen Baugruppe,
FIG. 4 eine Prinzipskizze eines Regelungsschemas gemäß einem Beispiel,
FIG. 5 eine Visualisierung einer Winkelabhängigkeit einer Kommutierungsmatrix,
FIG. 6 eine Prinzipskizze eines Regelungsschemas gemäß einem weiteren Beispiel,
FIG. 7A und FIG. 7B Draufsichten eines Teils der elektrischen Baugruppe nach FIG. 1A in verschiedenen Fehlerzuständen,
FIG. 8 ein Diagramm, das die Anzahl steuerbarer Freiheitsgrade für verschiedene elektrische Baugruppen in einem Fehlerzustand zeigt,
FIG. 9 eine Visualisierung der Auswirkung eines Fehlerzustands auf die Winkelabhängigkeit einer Kommutierungsmatrix,
FIG. 10A bis FIG. 10D Draufsichten jeweiliger Teile von elektrischen Baugruppen nach weiteren Beispielen,
FIG. 11 ein beispielhaftes Messergebnis für die Bestimmung und Reduktion von Querabhängigkeiten,
FIG. 12A einen Längsschnitt durch eine elektrische Baugruppe nach einem weiteren Beispiel,
FIG. 12B einen Querschnitt durch eine der Ebenen A--A, C--C in FIG. 12A,
FIG. 12C einen Querschnitt durch die Ebene B--B in FIG. 12A,
FIG. 13 ein Diagramm, das die Anzahl steuerbarer Freiheitsgrade für verschiedene weitere elektrische Baugruppen zeigt,
FIG. 14 ein Herzunterstützungssystem mit einer Blutpumpe (im Längsschnitt) und einer Steuereinheit,
FIG. 15A einen Längsschnitt durch eine Blutpumpe eines Herzunterstützungssystems nach einem weiteren Beispiel,
FIG. 16 einen Längsschnitt eines Lüfters,
FIG. 17 einen Längsschnitt einer Stabilisationsvorrichtung,
FIG. 18 einen Längsschnitt eines optischen Abbildungssystems mit einer Stabilisationsvorrichtung nach einem weiteren Beispiel.

Wiederkehrende und ähnliche Merkmale in den Zeichnungen sind mit identischen oder ähnlichen Bezugszeichen versehen. Diese können teilweise ausgelassen werden, wenn die entsprechenden Merkmale bereits in einer anderen Zeichnung gezeigt und im Zusammenhang mit dieser beschrieben werden oder wenn sie mit Bezug auf eine Zeichnung nicht erwähnt werden.

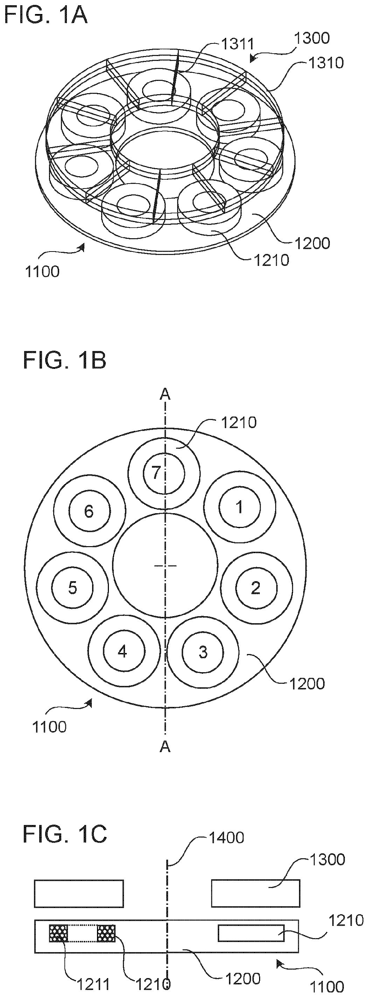

Die in FIG. 1A bis FIG. 1C dargestellte elektrische Baugruppe 1100 umfasst eine stationäre Komponente 1200 und eine aktuierbare Komponente 1300 (letztere ist in FIG. 1A zur besseren Sichtbarkeit der darunterliegenden Komponenten durchsichtig dargestellt).

Die stationäre Komponente 1200 umfasst eine Mehrzahl von Spuleneinheiten 1210, wobei jede der Mehrzahl von Spuleneinheiten 1210 zum Erzeugen eines variablen Magnetfelds ansteuerbar ist. Die aktuierbare Komponente 1300 ist relativ zu der stationären Komponente 1200 beweglich und umfasst eine Magnetanordnung 1310.

Die aktuierbare Komponente 1300 ist im gezeigten Beispiel bezüglich der stationären Komponente 1200 in sechs linear unabhängigen Freiheitsgraden beweglich gelagert und insbesondere um eine Rotationsachse 1400 rotierbar, also im Sinne eines Elektromotors aktuierbar (zum Beispiel für ein Herzunterstützungssystem oder eine andere der in dieser Anmeldung offenbarten Anwendungen). Die stationäre Komponente 1200 nimmt dabei also die Rolle eines Stators ein, die aktuierbare Komponente 1300 die Rolle eines Rotors. Im Folgenden werden diese Komponenten häufig entsprechend diesem Beispiel als Stator und Rotor bezeichnet, wenngleich die Diskussion in vielen Fällen auch auf andere Beispiele anwendbar ist.

Der Motor im gezeigten Beispiel ist ein Axialflussmotor (auch als AFM abgekürzt) oder könnte beispielsweise eine Axialfluss-Komponente eines Elektromotors im oben erwähnten Sinne bilden.

Jede einzelne der Mehrzahl von Spuleneinheiten 1210 ist zum Erzeugen des jeweiligen variablen Magnetfelds derart eingerichtet ist, dass durch Wechselwirkung des jeweiligen variablen Magnetfelds mit der Magnetanordnung 1310 Kräfte und/oder Momente bezüglich mehrerer Freiheitsgrade auf die aktuierbare Komponente 1300 wirken, und zwar so, dass durch Ansteuern einer beliebigen Kombination von höchstens sechs der Mehrzahl von Spuleneinheiten 1210 eine Position und/oder Bewegung der aktuierbaren Komponente 1300 relativ zu der stationären Komponente 1200 in den sechs linear unabhängigen Freiheitsgraden steuerbar ist (also die oben definierte Auswahleigenschaft erfüllt ist).

Im gezeigten Beispiel umfasst die stationäre Komponente 1200 sieben (in FIG. 1B mit den Zahlen 1 bis 7 durchnummerierte) Spuleneinheiten 1210, die jeweils eine Spule (im allgemeinen Fall eine oder mehrere Spulen) mit Windungen 1211 (im allgemeinen Fall mit einer oder mehreren jeweiligen Windungen) umfassen und sieben Motorphasen des Elektromotors entsprechen. Die Spuleneinheiten 1210 sind in diesem Beispiel koplanar und äquidistant um die Rotationsachse 1400 herum angeordnet. Die Magnetanordnung 1310 umfasst in diesem Beispiel fünf Polpaare 1311. Im Folgenden werden Spule und Spuleneinheit mitunter entsprechend diesem Beispiel synonym verwendet, wenngleich die Diskussion in vielen Fällen auch auf andere Beispiele (etwa mit mehr als einer Spule pro Spuleneinheit etc.) anwendbar ist.

Jede einzelne der Mehrzahl von Spuleneinheiten 1210 ist hierbei so verschaltet, dass sie unabhängig von jeder anderen der Mehrzahl von Spuleneinheiten 1210 zum Erzeugen des jeweiligen variablen Magnetfelds ansteuerbar ist. Insbesondere sind die Spuleneinheiten 1210 nicht über gemeinsame nicht angesteuerte Eck- oder Sternpunkte verschaltet, was die Anzahl unabhängig voneinander ansteuerbarer (hier auch kurz: unabhängiger) Spuleneinheiten 1210 verringern würde.

Die elektrische Baugruppe 1100 erfüllt beispielhaft die genannte Auswahleigenschaft, die jedoch auch durch andere Anordnungen erfüllt werden kann. Im Folgenden sollen Prinzipien einer Steuerbarkeitsanalyse diskutiert werden, anhand derer eine entsprechend geeignete Zahl, Anordnung, Form und/oder Verschaltung der Spuleneinheiten 1210 festgelegt werden kann.

Um herauszufinden, welche Freiheitsgrade in einer Maschine unabhängig voneinander steuerbar sind, kann die Maschine zunächst wie folgt mit einer Matrix-Gleichung beschrieben werden: $\left(\begin{matrix}{F}_{x} \\ {F}_{y} \\ {F}_{z} \\ {M}_{x} \\ {M}_{y} \\ {M}_{z}\end{matrix}\right) = FM = {T}_{m} ⋅ {I}_{p} = \left[\begin{matrix}{c}_{Fxi 1} & {c}_{Fxi 2} & … & {c}_{Fxip} \\ {c}_{Fyi 1} & {c}_{Fyi 2} & … & {c}_{Fyip} \\ {c}_{Fzi 1} & {c}_{Fzi 2} & … & {c}_{Fzip} \\ {c}_{Mxi 1} & {c}_{Mxi 2} & … & {c}_{Mxip} \\ {c}_{Myi 1} & {c}_{Myi 2} & … & {c}_{Myip} \\ {c}_{Mzi 1} & {c}_{Mzi 2} & … & {c}_{Mzip}\end{matrix}\right] ⋅ \left(\begin{matrix}{i}_{1} \\ {i}_{2} \\ … \\ {i}_{p}\end{matrix}\right) .$

Dabei sind:
FM ein Kraft-Moment-Vektor, bestehend aus den auf den Rotor bezüglich eines kartesischen Koordinatensystems (mit Positionskoordinaten x, y, z) wirkenden Kräften Fₓ, F_{y}, F_{z} und den Momenten Mₓ, M_{y}, M_{z};
Iₚ ein Phasenstromvektor mit den Phasenströmen i₁ bis iₚ;
p die Anzahl der Phasen;
Tₘ (auch als Tm geschrieben) eine Verknüpfungsmatrix der Dimension 6 x p.

Ohne Beschränkung der Allgemeinheit wird hier die z-Achse als Hauptantriebsachse gewählt, M_{z} ist damit das Hauptantriebsmoment. Bei den meisten Motoren ist die Drehung um die Hauptantriebsachse nicht beschränkt und muss damit für eine Lageregelung nicht geregelt werden.

Tₘ ist im Allgemeinen abhängig von der Position und Orientierung des Rotors: ${T}_{m} = {T}_{m _ f} \left(x,y,x, {θ}_{x}, {θ}_{y}, {θ}_{z}\right) ,$ wobei T_{m_f} eine Generatorfunktion der Verknüpfungsmatrix mit den Parametern der Rotorposition x,y,z, mit x der Rotorposition in der x-Achse (y, z entsprechend) und der Rotororientierung θₓ, θ_{y}, θ_{z}, mit θₓ (Theta-x) der Rotation um die x-Achse (y, z entsprechend), darstellt. An dieser Stelle wird mit Position die translatorische Position und mit Orientierung die rotatorische Position bezeichnet, im Allgemeinen kann jedoch im Sprachgebrauch dieser Anmeldung der Ausdruck Position oder Lage auch zusammenfassend für die translatorische und rotatorische Position verwendet werden. Bei nicht aktiv magnetisch gelagerten Antriebssystemen wird oftmals ebenfalls von einer Rotorposition gesprochen. Dabei handelt es sich allerdings im Allgemeinen um die rotatorische Rotorposition in Drehrichtung. Beispielsweise wird der zur Kommutierung herangezogene Rotorwinkel oftmals als Rotorposition Beschrieben. Damit unterscheidet sich die Rotorposition in vielen Darstellungen von der hier dargelegten allgemeinen sechsdimensionalen Rotorposition.

T_{m_f} kann durch Simulation, beispielsweise nach der Finite-Elemente-Methode, bestimmt werden oder auch durch analytische Berechnung ermittelt werden. Für eine analytische Berechnung von T_{m_f} kann es sinnvoll sein, Vereinfachungen am Modell vorzunehmen. Mögliche Vereinfachungen sind die Abbildung von ausgedehnten Magneten durch einen einzelnen magnetischen Dipol oder eine Ansammlung von magnetischen Dipolen. Spulen können ebenso als elektrisch steuerbare magnetische Dipole oder als wenige Leiterzüge der Dicke 0 approximiert werden.

Für die vorliegende Analyse wird angenommen, dass der Bewegungsraum in x, y, x, θₓ, θ_{y} beschränkt ist. Da die Drehung um die z-Achse, θ_{z}, die Antriebsachse darstellt, muss hier der vollständige Winkelbereich von 0° bis 360° abgedeckt werden. Bei höheren Rotor-Polpaarzahlen kann der Bereich auf die Periode (0°-360°)/Polpaarzahl begrenzt werden, sofern die Polpaare rotationssymmetrisch angeordnet sind. Für die begrenzten Freiheitsgrade kann zunächst angenommen werden, dass Tₘ sich bei Verschiebung nur unwesentlich ändert, da auch hier eine Vorzugsposition besteht. Dadurch wird die Generatorfunktion vereinfacht zu ${T}_{m _ f} \left(x, y, z, {θ}_{x}, {θ}_{y}, {θ}_{z}\right) ∼ {T}_{m _ f} \left(x=0, y=0, z=0, {θ}_{x}=0, {θ}_{y}=0, {θ}_{z}\right) = {T}_{m _ f} \left({θ}_{z}\right) .$

Die Steuerbarkeit für jede Rotorposition kann durch Berechnung des Matrix-Rangs (rank) der Tₘ-Matrix ermittelt werden: ${n}_{DOF} = Minimum \left({n}_{DOF}\left({θ}_{z}\right)\right) = Minimum \left(rank\left({T}_{m _ f}\left({θ}_{z}\right)\right)\right) , für alle {θ}_{z} ,$ mit n_{DOF} : Anzahl der unabhängig steuerbaren Freiheitsgrade (DOF steht dabei für "degree of freedom" = Freiheitsgrad).

Für n_{DOF} = 6 ist der Rotor vollständig steuerbar. Das heißt, die Position in x,y,z und die Rotation θₓ, θ_{y}, θ_{z} sind einzeln, aber auch in beliebigen Kombinationen, steuerbar.

Für n_{DOF} < 6 sind nur n_{DOF} verschiedene Linearkombinationen von Fₓ, F_{y}, F_{z}, Mₓ, M_{y} und M_{z} steuerbar.

Für einen beispielhaften dreiphasigen Axialflussmotor, wie er aus dem Stand der Technik bekannt ist, ergibt sich n_{DOF} = 2, da nur F_{z} und M_{z} unabhängig gesteuert werden können. Da in einen dreiphasigen Motor mit Stern- oder Dreieckschaltung nur zwei Ströme unabhängig voneinander eingeprägt werden können, ist der Informationsgehalt im Stromvektor auch nur zweidimensional. Quasistatisch kann n_{DOF} nie größer sein als der Informationsgehalt des Stromvektors.

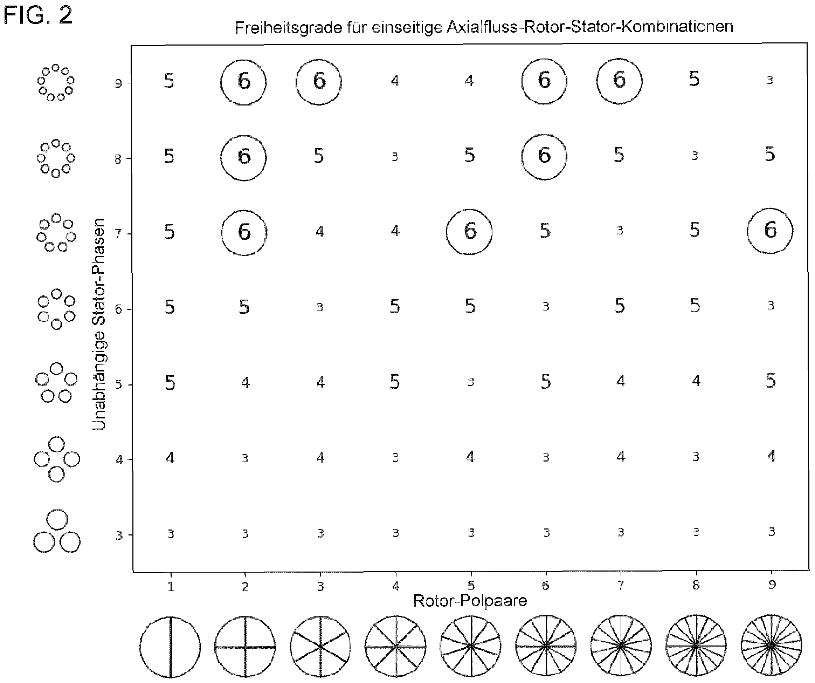

Zur Untersuchung weiterer Axialflussmotor-Topologien kann ein T_{m_f}-Modell erstellt werden, welches eine beliebige Anzahl an Spulen und Polpaaren abbilden kann. Durch eine Steuerbarkeitsanalyse kann basierend darauf eine Übersicht über die Steuerbarkeit für verschiedene Kombinationen von Spulen und Polpaaren erzeugt werden, wie in FIG. 2 gezeigt.

Mit drei, vier oder fünf unabhängig verschalteten Spulen lassen sich, wie in FIG. 2 ersichtlich, mit der geeigneten Polpaarzahl auch ebenso viele Freiheitsgrade gleichzeitig (quasistatisch) steuern, beispielsweise mit der Polpaarzahl 1. Mit einigen Kombinationen, wie beispielsweise vier Spulen und zwei Polpaaren ist die Steuerbarkeit jedoch eingeschränkt und die Anzahl gleichzeitig regelbarer Freiheitsgrade reduziert. Dies lässt sich auf Symmetrien zwischen Spulen, Polpaaren und der Geometrie zurückführen. Diese Symmetrie zwischen Magnetfeld und Spulenpositionen sorgt dafür, dass die jeweilig von den Einzelspulen erzeugbaren Kraft-Moment-Vektoren KMₙ in ähnliche oder sogar gleiche Richtung zeigen und damit linear abhängig sind. Die lineare Abhängigkeit kann dann dazu führen, dass trotz 6 oder mehr steuerbaren Strömen in Kombination weniger Freiheitsgrade steuerbar sind. Es kann dabei auch eine einfache Redundanz zwischen zwei Spulen entstehen. Einfache Redundanz heißt, dass eine Spule durch eine andere ersetzt werden kann, beide Spulen in Kombination aber nicht mehr Aktorrichtungen ermöglichen als eine Einzelspule. Beispielsweise ist die die Anzahl gleichzeitig regelbarer Freiheitsgrade nie größer als drei, wenn die Anzahl der Spulen der Anzahl der Polpaare entspricht.

Besonders bemerkenswert ist, dass es für sechs äquidistant angeordnete Spulen keine Polpaarzahl mit 6 Freiheitsgraden gibt. Hier limitiert die Symmetrie des Stators die Regelbarkeit.

Sieben ist die kleinste Anzahl an äquidistant radial verteilten Spulen mit 6 steuerbaren Freiheitsgraden (für 2, 5 oder 9 Polpaare). Die Konfiguration mit 7 Spulen und 5 Polpaaren entspricht der in Fig. 1A, 1B oder 1C dargestellten elektrischen Baugruppe.

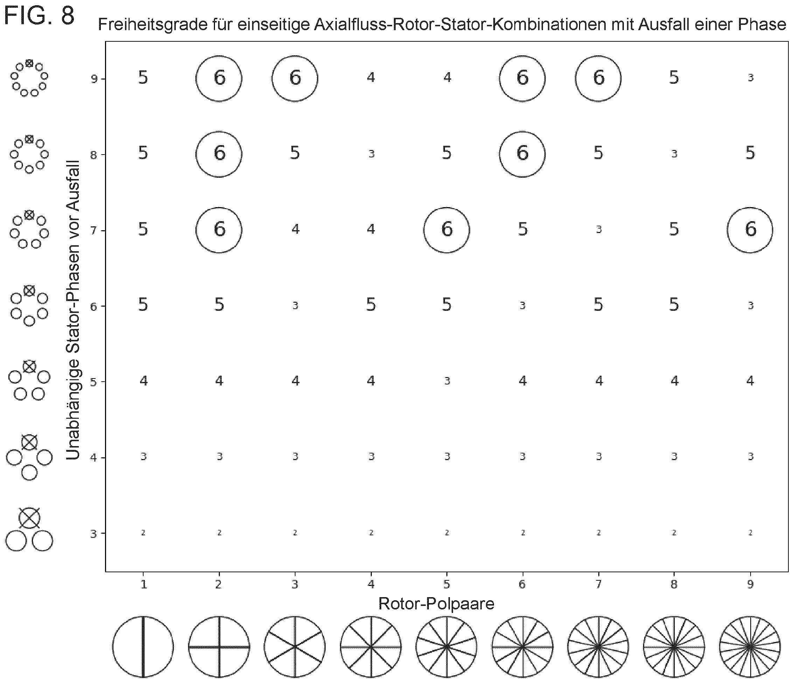

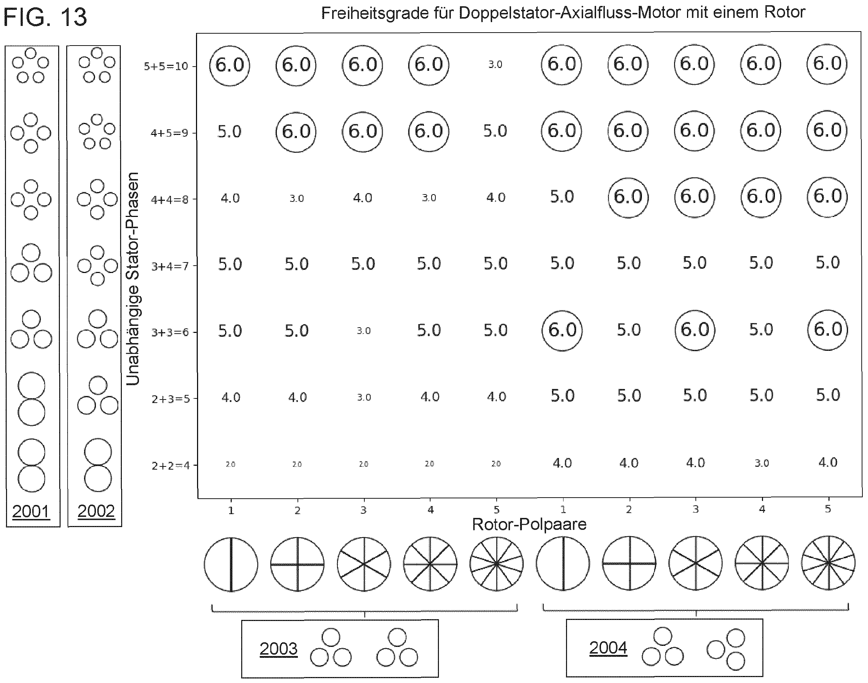

Alle quasistatisch voll steuerbaren Konfiguration sind in Fig. 2 (sowie in Fig. 8 und Fig. 13) eingekreist und als besonders vorteilhafte Konfigurationen im Sinne der Anmeldung zu verstehen.

Die Besonderheit eines 6-phasigen AFM, dass dieser trotz 6 unabhängig einprägbarer Ströme nur 5 unabhängig steuerbare Freiheitsgrade besitzt, soll an dieser Stelle mit einem Rechenbeispiel veranschaulicht werden. Für das Beispiel wird die Tₘ-Matrix für einen AFM mit einem Rotor mit 5 Polpaaren berechnet, da anschließend für denselben Rotor die volle Steuerbarkeit mit 7 Spulen demonstriert werden soll.

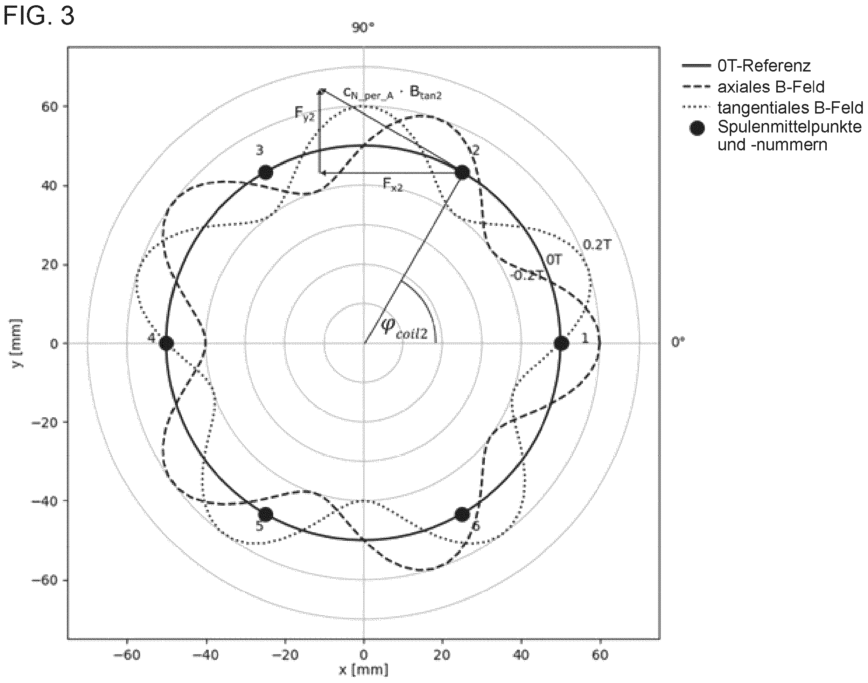

Es wird angenommen, dass der Rotor sich parallel zum Stator befindet. Rotormagnete der Magnetanordnung des Rotors werden, beispielsweise durch eine Halbach-Anordnung, so gewählt, dass das von den Rotormagneten auf Höhe der Spulen erzeugte B-Feld in axialer Richtung kosinusförmig und in tangentialer Richtung sinusförmig variiert. Da die einzelnen Spulen wesentlich kleiner sind als der Rotor, werden diese hier als stromgesteuerte Dipole approximiert. Damit ergibt sich vor Berechnung der Kräfte und Momente die in FIG. 3 gezeigte Relation zwischen den Dipolen an den Spulenpositionen und den axialen und tangentialen Magnetfeldern. Wenn der Rotor sich dreht, dann verschieben sich die B-Felder, während die Spulen verharren.

An den Stellen der Spulen lässt sich nun jeweils das lokale axiale und tangentiale Magnetfeld bestimmen. Da die Spule als stromabhängiger Dipol modelliert wird, erzeugt ein Spulenstrom eine zum axialen Rotorfeld proportionale Axialkraft und eine ebenfalls zum tangentialen Rotorfeld proportionale Tangentialkraft an den Stellen des Rotors in unmittelbarer Nähe der Dipole. Diese Kräfte wirken auf die Spulen und in umgekehrter Richtung auch auf den Rotor.

Aus den 6 axialen und tangentialen Kräften können anschließend die auf den Schwerpunkt des Rotors bezogene Kräfte in x, y und z-Richtung und Momente um x, y und z-Achse berechnet werden. Der Schwerpunkt kann dabei je nach Anwendungsfall der Massenschwerpunkt oder ein magnetischer Lagerschwerpunkt (oder eine Kombination aus beiden) sein. Die hier zu betrachtende Maschine hat keine passiven magnetischen Lager, daher wird Tₘ auf den Massenschwerpunkt bezogen und wie folgt berechnet: ${T}_{m} = {c}_{N _ per _ A} \left[\begin{matrix}{B}_{tan 1} ⋅ sin \left({φ}_{coil 1}\right) & … & {B}_{tan 6} ⋅ sin \left({φ}_{coil 6}\right) \\ {B}_{tan 1} ⋅ cos \left({φ}_{coil 1}\right) & … & {B}_{tan 6} ⋅ cos \left({φ}_{coil 6}\right) \\ {B}_{ax 1} & … & {B}_{ax 6} \\ {r}_{c} ⋅ {B}_{ax 1} ⋅ sin \left({φ}_{coil 1}\right) & … & {r}_{c} ⋅ {B}_{ax 6} ⋅ sin \left({φ}_{coil 6}\right) \\ {r}_{c} ⋅ {B}_{ax 1} ⋅ cos \left({φ}_{coil 1}\right) & … & {r}_{c} ⋅ {B}_{ax 6} ⋅ cos \left({φ}_{coil 6}\right) \\ {r}_{c} ⋅ {B}_{tan 1} & … & {r}_{c} ⋅ {B}_{tan 6}\end{matrix}\right]$ $\left(Matrixeinträge:\begin{matrix}{F}_{x 1 .. 6} \\ {F}_{y 1 .. 6} \\ {F}_{z 1 .. 6} \\ {M}_{x 1 .. 6} \\ {M}_{y 1 .. 6} \\ {M}_{z 1 .. 6}\end{matrix}\right) .$

Dabei sind:
*c_{N_per_A}*: Spulenkraft pro magnetische Flussdichte in Newton pro Tesla;
*Bₜₐₙᵢ* : Magnetische Flussdichte der i-ten Spule in tangentialer Richtung in Tesla;
*Bₐₓᵢ* : Magnetische Flussdichte der i-ten Spule in axialer Richtung in Tesla;
*φ_{coili}* : Winkelposition der i-ten Spule in Rad;
*r_{c}* : Radius eines Kreises, auf dem die Spulen beziehungsweise Dipole liegen, in Meter.
*φ*_{*coil*2}, sowie die Tₘ-Matrixeinträge Fₓ₂ und F_{y2} sind zur Veranschaulichung in FIG. 3 eingezeichnet. Die Kräfte der weiteren Spulen werden äquivalent bestimmt, und die axialen Kräfte Zeigen von den Spulenpositionen aus in die Abbildung hinein.

Die Tₘ-Matrix ist damit für diesen konkreten Fall: $\begin{matrix}\left(\begin{matrix}{F}_{x} \\ {F}_{y} \\ {F}_{z} \\ {M}_{x} \\ {M}_{y} \\ {M}_{z}\end{matrix}\right) = FM = {T}_{m _ 6 coils} ⋅ {I}_{p} = \\ \left[\begin{matrix}0 & 0.38 & - 0.38 & 0 & - 0.38 & 0.375 \\ 0 & 0.22 & 0.22 & 0 & - 0.22 & - 0.22 \\ 0.5 & - 0.25 & - 0.25 & 0.5 & - 0.25 & - 0.25 \\ 0 & - 0.01 & - 0.01 & 0 & 0.02 & 0.01 \\ 0.03 & - 0.006 & 0.006 & - 0.025 & 0.006 & - 0.005 \\ 0 & 0.02 & - 0.02 & 0 & 0.02 & - 0.02\end{matrix}\right] ⋅ \left(\begin{matrix}{i}_{1} \\ {i}_{2} \\ {i}_{3} \\ {i}_{4} \\ {i}_{5} \\ {i}_{6}\end{matrix}\right) .\end{matrix}$

Die Anzahl der unabhängig steuerbaren Freiheitsgrade entspricht dem Rang der Tₘ-Matrix. Dieser kann numerisch berechnet werden und ergibt 5, also weniger als die Größe der hier quadratischen Tₘ-Matrix.

Die Steuerbarkeit lässt sich besonders anschaulich darstellen, wenn man Tₘ mit einer QR-Zerlegung durch das Produkt einer quadratischen Matrix und einer rechten Dreiecksmatrix darstellt: $\begin{matrix}{T}_{m _ 6 coils} = {Q}_{6 coils} ⋅ {R}_{6 coils} \\ = {Q}_{6 coils} ⋅ \left[\begin{matrix}- 0.5 & 0.25 & 0.25 & - 0.5 & 0.25 & 0.25 \\ 0 & - 0.433 & 0.22 & 0.0007 & 0.43 & - 0.21 \\ 0 & 0 & - 0.37 & 0.003 & - 0.0002 & 0.38 \\ 0 & 0 & 0 & 0.05 & - 0.025 & - 0.02 \\ 0 & 0 & 0 & 0 & 0.04 & - 0.04 \\ 0 & 0 & 0 & 0 & 0 & 0\end{matrix}\right] .\end{matrix}$

An der R-Matrix lassen sich die linear unabhängigen Zeilen direkt ablesen. Durch den Eintrag des von Null verschiedenen Wertes auf der Diagonalen von R ist der Rang und damit die Steuerbarkeit reduziert.

Stellt man analog Tₘ für den 7-Phasigen AFM mit demselben Rotor mit 5 Polpaaren (also etwa das in FIG. 1A bis FIG. 1C gezeigte Beispiel) auf, dann ergibt sich folgende Tₘ- und R-Matrix: $\begin{matrix}{T}_{{m}_{7 coils}} = {Q}_{7 coils} ⋅ {R}_{7 coils} \\ = {Q}_{7 coils} ⋅ \left[\begin{matrix}- 0.5 & 0.11 & 0.45 & - 0.32 & - 0.31 & 0.45 & 0.11 \\ 0 & - 0.49 & 0.14 & - 0.09 & 0.35 & 0.19 & - 0.11 \\ 0 & 0 & - 0.17 & - 0.37 & 0.17 & - 0.1 & 0.46 \\ 0 & 0 & 0 & - 0.11 & 0.02 & - 0.004 & 0.09 \\ 0 & 0 & 0 & 0 & 0.04 & - 0.006 & - 0.04 \\ 0 & 0 & 0 & 0 & 0 & 0.04 & - 0.04\end{matrix}\right]\end{matrix}$

Hier haben alle Diagonaleinträge einen signifikant von Null verschiedenen Wert und die volle Steuerbarkeit ist damit gegeben.

Anstatt der QR-Zerlegung kann auch eine Singulärwertzerlegung von Tₘ durchgeführt werden. Dabei erhält man dann nicht nur die Anzahl der Freiheitsgrade, sondern mit den Eigenvektoren auch ein Hilfsmittel, um die zu messenden oder zu regelnden Achsen der Freiheitsgrade, insbesondere bei einer unterbestimmten Regelung, zu wählen. Die zu messenden Achsen oder zu regelnden Achsen oder die Achsen des Koordinatensystems müssen dabei nicht zwangsläufig zusammenfallen und können beliebig gewählt und ineinander umgerechnet werden.

Weitere Eigenschaften der in FIG. 1A bis FIG. 1C gezeigten beispielhaften elektrischen Baugruppe 1100 (und weiterer Anordnungen entsprechender Steuerbarkeit) ergeben sich wie folgt.

Zum einen ergibt sich, dass zu jeder vorgegebenen Spuleneinheit 1210 der Mehrzahl von Spuleneinheiten 1210 eine erste Position der aktuierbaren Komponente 1300 und eine zweite Position der aktuierbaren Komponente 1300 existieren,
so dass in der ersten Position durch Wechselwirkung der Magnetanordnung 1310 mit dem mittels der vorgegebenen Spuleneinheit 1210 erzeugbaren variablen Magnetfeld eine Kraft und/oder ein Moment erzeugbar ist, das bezüglich eines ersten Freiheitsgrades der sechs linear unabhängigen Freiheitsgrade auf die aktuierbare Komponente 1300 wirkt und
so dass in der zweiten Position durch Wechselwirkung der Magnetanordnung 1310 mit dem mittels der vorgegebenen Spuleneinheit 1210 erzeugbaren variablen Magnetfeld eine Kraft und/oder ein Moment erzeugbar ist, das bezüglich eines zweiten Freiheitsgrades der sechs linear unabhängigen Freiheitsgrade auf die aktuierbare Komponente 1300 wirkt.

Zum anderen ergibt sich, dass zu jedem vorgegebenen der sechs Freiheitsgrade mindestens eine erste Spuleneinheit 1210 der Mehrzahl von Spuleneinheiten 1210 und eine zweite Spuleneinheit 1210 der Mehrzahl von Spuleneinheiten 1210 existieren,
so dass durch Wechselwirkung der Magnetanordnung 30 mit dem mittels jeder einzelnen der ersten Spuleneinheit 1210 und der zweiten Spuleneinheit 1210 erzeugbaren variablen Magnetfeld eine Kraft und/oder ein Moment erzeugbar ist, das bezüglich des vorgegebenen Freiheitsgrades auf die aktuierbare Komponente 1300 wirkt.

Im Folgenden soll nun auf Steuerverfahren zum Ansteuern einer elektrischen Baugruppe 1100, einschließlich des vorstehend diskutierten Beispiels, eingegangen werden. Solche Steuerverfahren können insbesondere in einer Steuereinheit zum Ansteuern einer elektrischen Baugruppe der beschriebenen Art implementiert sein, wobei die Steuereinheit eingerichtet ist zum Ansteuern einer elektrischen Baugruppe der hier diskutierten Art.

Allgemein umfasst das Steuerverfahren:
Bestimmen einer Mehrzahl von Steuervorgaben zum Ansteuern jeder der Mehrzahl von Spuleneinheiten,
Steuern und/oder Regeln einer Position und/oder Bewegung der aktuierbaren Komponente relativ zu der stationären Komponente durch Beaufschlagen der Mehrzahl von Spuleneinheiten mit einem Signal gemäß der bestimmten Mehrzahl von Steuervorgaben.

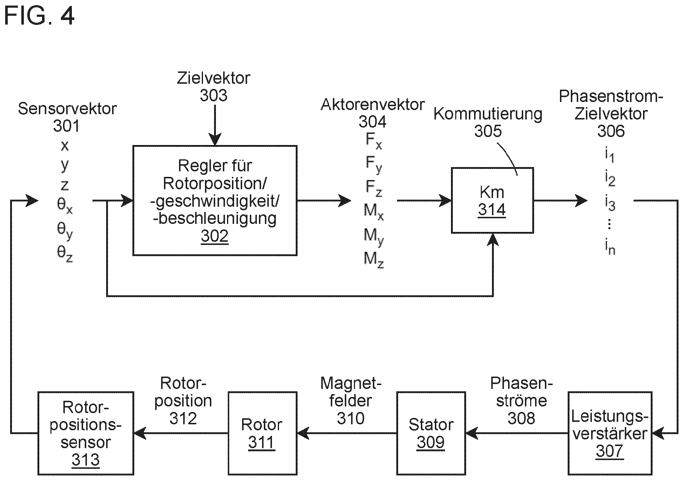

FIG. 4 zeigt ein Regelungsschema, dass ein Beispiel dieses Steuerverfahrens implementiert. Dabei wird die Mehrzahl von Steuervorgaben als Steuervorgabenvektor (Phasenstrom-Zielvektor 306) unter Verwendung einer Matrixmultiplikation einer Kommutierungsmatrix (Kₘ) 314, die insbesondere von der Position der aktuierbaren Komponente 1300 abhängt, mit einem Kraft-Moment-Vektor (Aktorenvektor 304) bestimmt. Die Kommutierungsmatrix 314 soll im Folgenden näher diskutiert werden.

Um einen Motor zu steuern oder zu regeln ist die oben diskutierte Tₘ nicht direkt geeignet, da über die Tₘ-Matrix die Rotorkräfte und -momente aus den Phasenströmen berechnet werden. Für eine Regelung der Rotorposition werden allerdings Sollkräfte und -momente mithilfe von Reglern bestimmt. Anschließend müssen die notwendigen Phasenströme für diese Sollkräfte und - momente berechnet werden. Bei symmetrischen Tₘ₋Matrizen kann hierfür direkt die inverse Tₘ Matrix verwendet werden: $\begin{matrix}FM = {T}_{m} ⋅ {I}_{p} \\ ⇒ {I}_{p} = {{T}_{m}}^{- 1} ⋅ FM ,\end{matrix}$ mit Tₘ⁻¹: Inverse von T_{M}.

Dafür muss Tₘ invertierbar sein. Dies setzt eine quadratische Matrix mit vollem Rang voraus.

Für nicht quadratische Matrizen ist die Inverse allerdings nicht definiert. Dies ist jedoch für alle Motoren mit 7 oder mehr unabhängigen Phasen der Fall. Hat Tₘ dort einen ausreichenden Rang von 6, dann kann es mehrere Lösungen für die Gleichung ${{T}_{m}}^{- 1} = {K}_{m} = \frac{{I}_{p}}{FM} = {f}_{Ip} \left(FM\right)$ geben. Da Tₘ⁻¹=Kₘ bestimmt, wie die Phasen bestromt werden sollen, wird Kₘ (auch als Km geschrieben) als Kommutierungsmatrix (auch Kommutierungsvorschrift oder Kommutierungsfunktion) bezeichnet.

Zur Laufzeit ist dann lediglich eine Matrixmultiplikation erforderlich, um für beliebige Rotorkräfte oder -momente den Phasenstrom-Zielvektor zu berechnen: ${I}_{p} = {K}_{m} ⋅ FM .$

Um aus den vielen möglichen Lösungen für Kₘ die optimale Lösung zu bestimmen, kann vorgesehen sein, dass die Kommutierungsmatrix auf Grundlage der Verknüpfungsmatrix Tₘ unter Berücksichtigung mindestens eines Optimierungskriteriums, beispielsweise einer Minimierung einer Verlustleistung und/oder einer Maximierung einer Steuerbarkeit, bestimmt wird.

Bei Phasen mit identischem Spulenwiderstand ist die Verlustleistung $P = {\sum }_{n = 1}^{p} {{i}_{n}}^{2} ⋅ R = R ⋅ {\sum }_{n = 1}^{p} {{i}_{n}}^{2}$ mit
P: Gesamtverlustleistung in allen Spulen,
iₙ: Phasenstrom der n-ten Phase,
R: Phasenwiderstand.

Bei gleichen Phasenwiderständen muss zur Minimierung der Verlustleistung also die Summe der Quadrate der Phasenströme minimiert werden. Dies entspricht auch der Minimierung der geometrischen Summe der Phasenströme, ${I}_{geo} = \sqrt{{\sum }_{n = 1}^{p} {{i}_{n}}^{2}} ,$ und damit auch der Matrix-2-Norm. Die Minimierung der 2-Norm bei der Invertierung einer nicht quadratischen Matrix ist in der Numerik als die Pseudo-Inverse bekannt. Diese wird dargestellt als hochgestelltes "+". Kₘ kann damit für jede Rotorposition bestimmt werden zu ${K}_{m} \left(x, y, z, {θ}_{x}, {θ}_{y}, {θ}_{z}\right) = Pseudoinverse \left({T}_{m}\left(x, y, z, {θ}_{x}, {θ}_{y}, {θ}_{z}\right)\right) = {{T}_{m}}^{+} ,$ und für den hier vorgestellten konkreten Fall ${K}_{m} \left({θ}_{z}\right) = {T}_{m} {\left({θ}_{z}\right)}^{+} .$

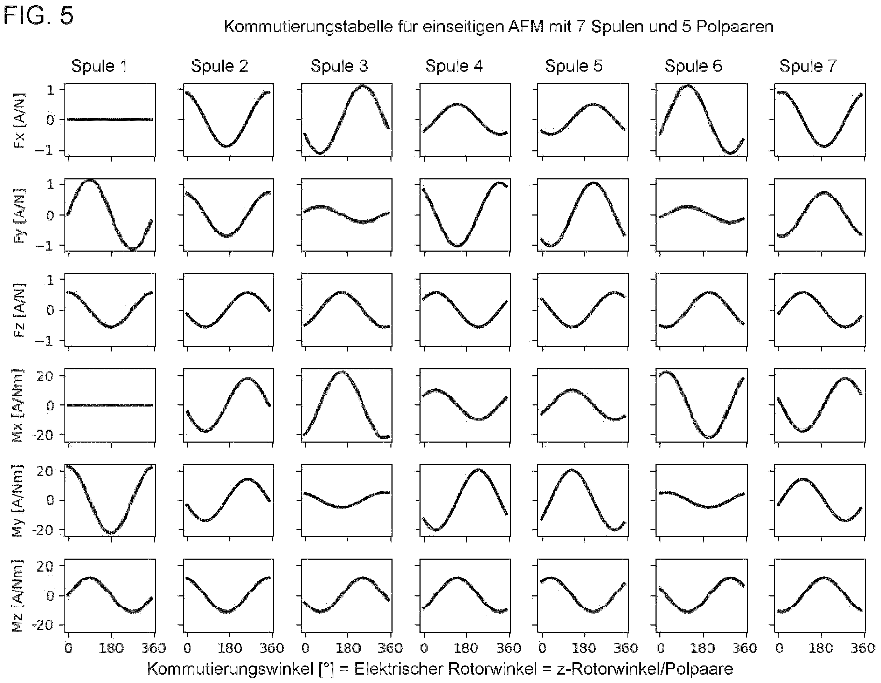

Stellt man Kₘ(θ_{z}) für den Axialflussmotor mit sieben Spulen und 5 Polpaaren auf, dann ergibt sich für die gesamte Winkelperiode von 0°-360°/Polpaare=72° ein Kₘ wie in FIG. 5 dargestellt.

Kₘ kann direkt, etwa als Lookup-Table, im Speicher des Motorreglers hinterlegt werden. Bei den Vereinfachungen der Magnete oder Spulen zu magnetischen Dipolen ist der Verlauf jeder Komponente von Kₘ sinusförmig. Daher kann jede Kₘ-Komponente für beliebig viele Rotorwinkel durch eine Sinuskurve mit Amplitude und Phasenverschiebung dargestellt werden und der Verlauf der Kommutierungsmatrix über den Kommutierungswinkel somit speichereffizient hinterlegt werden.

Für Minimierungen, die sich von der 2-Norm unterscheiden, können die Zeilen von Kₘ (entspricht auch den Zeilen in FIG. 5) für jeden Freiheitsgrad und jede Rotorposition einzeln mit iterativen Verfahren minimiert werden. Das Minimierungsverfahren "konjugierte Gradienten" generiert hierbei Ergebnisse in der Nähe der Pseudo-Inversen. Zur Verbesserung der Konvergenz des Minimierungsverfahrens kann zusätzlich die Information über die Form jeder Matrix-Komponente über den Rotorwinkel genutzt werden. In diesem Beispiel ist der Verlauf sinusförmig mit einer definierten Amplitude sowie Phasenverschiebung und ist zudem mittelwertfrei. Da nun weniger Parameter mit dem Optimierungsverfahren optimiert werden müssen, ist dieses in der Regel stabiler.

Nun wird wieder auf FIG. 4 Bezug genommen. Um einen Rotor 309 in sechs Freiheitsgraden zu regeln, wird die Rotorposition in diesen Freiheitsgraden von einem Rotorpositionssensor 313 gemessen. Der Sensorvektor 301 mit den translatorischen Positionen x, y, z und den rotatorischen Positionen θₓ, θ_{y}, θ_{z} (Orientierung) kann dann einem Regler 302 zugeführt werden, welcher gleichzeitig auch einen Zielvektor erhält. Der Zielvektor 303 kann Positionen, optional auch Geschwindigkeiten sowie Beschleunigungen, vorgeben. Aufgabe des Reglers 302 ist es, Zielkräfte und -momente (Aktorenvektor 304) zu bestimmen, welche den Rotor zum Zielvektor bewegen.

Der Regler 302 kann als Kombination einzelner Regler für jeden Freiheitsgrad ausgeführt sein. Für die einzelnen Regler kommen alle gängigen Reglerstrukturen, beispielsweise PID-Regler, Lead-Lag-Regler, Fuzzy-Regler, adaptive Regler oder Extremwertregler in Betracht. Der Regler kann auch als mehrdimensionaler Regler, beispielsweise modellbasierter Regler, ausgeführt sein. Beobachter wie Luenberger Beobachter oder Kalman-Filter können mit einer Zustandsrückführung (State-Feedback) hier ebenfalls zum Einsatz kommen.

Basierend auf dem Aktorenvektor 304, bestehend aus Zielkräften und -momenten, wird anschließend eine Kommutierung 305 durchgeführt, also der Phasenstrom-Zielvektor 306 berechnet, insbesondere - wie bereits beschrieben - durch eine Matrixmultiplikation mit der Kommutierungsmatrix Km 314. Die Matrix Km ist abhängig von der Rotorposition. Abhängig von den Signalen (Sensorvektor) 301 eines Rotorpositionssensors 313, der eine Rotorposition 312 misst, wird Km aus einer Lookup-Tabelle ausgewählt oder neu berechnet. Jede Spalte der Kommutierungsmatrix Km enthält den Einheitsstromvektor, die Phasenstromkomponenten, welche notwendig sind um eine Einheit Kraft oder Drehmoment des jeweiligen Freiheitsgrads zu erzeugen. Die Matrixmultiplikation skaliert alle Einheitsstromvektoren mit der Kraft- oder Momentvorgabe (Aktorenvektor 304) und summiert alle Komponenten einer Phase auf, um die Komponenten des Phasenstrom-Zielvektors 306 zu berechnen.

Für eine quasistatische Regelung der Rotorposition kann der Phasenstrom-Zielvektor 306 direkt skaliert und durch eine Endstufe (hier: Leistungsverstärker 307), beispielsweise durch PWM-gesteuerte H-Brücken oder Halbbrücken als Spannung an die Motorphasen ausgegeben werden. Der sich darauf einstellende Phasenstrom 308 hängt vom Phasenwiderstand, der Phaseninduktivität und der induzierten Back-EMF ab. Der Phasenwiderstand limitiert den Phasenstrom 308 bei gegebener Phasenspannung. Sind Phaseninduktivität und Back-EMF gering, dann kann eine reine Spannungssteuerung des Stroms ausreichend sein. Die Back-EMF ist bei langsamen Bewegungen (einschließlich geringen Drehzahlen) gering. Die Induktivität ist bei eisenlosen Wicklungen besonders gering, daher sind diese Motoren für die Matrix-Kommutierung besonders geeignet.

Bei mittleren Drehzahlen kann der Einfluss der Induktivität durch eine Vorsteuerung, insbesondere durch einen Phasenoffset dθ_{z} bei der Bestimmung der Kommutierungsmatrix, kompensiert werden. Hierbei ist zu beachten, dass bei Mehrphasenmotoren die Gegeninduktivität signifikant sein kann und somit eine Stromänderung in einer Phase andere Phasen beeinflussen kann. In dreiphasigen Motoren können die Spulen oftmals so angeordnet werden, sodass sich die Gegeninduktionen durch die Verschaltung der Spulen in Stern- oder Dreieckschaltung nahezu aufhebt. Dieser Vorteil ist bei mehrphasigen Motoren nur bei bestimmten Stromkombinationen, aber nicht generell, gegeben. Der Einfluss der Back-EMF kann ebenfalls durch eine Vorsteuerung, in diesem Fall abgeleitet von der gemessenen Rotorposition und deren Änderungsraten, kompensiert werden. Die Endstufe kann zudem eine Phasenstrommessung und Stromregler für jede Phase umfassen.

Die in die Phasen des Stators 309 eingeprägten Phasenströme 308 erzeugen ihrerseits Magnetfelder 310, welche mit den Magnetfeldern der Magnetanordnung des Rotors 311 interagieren und somit Kräfte und Momente auf den Rotor 311 ausüben. Für eisenlose Wicklungen kann die komplette Krafterzeugung durch die Lorentzkraft berechnet werden.

Die Differenz zwischen den magnetischen Antriebskräften beziehungsweise - momenten und den Abtriebskräften beziehungsweise -momenten sowie der Rotorträgheitstensor bestimmen die Rotorbewegung.

Über den Rotorpositionssensor 313 wird die Rotorposition 312 in allen zu regelnden Freiheitsgraden gemessen und somit der Regelkreis geschlossen.

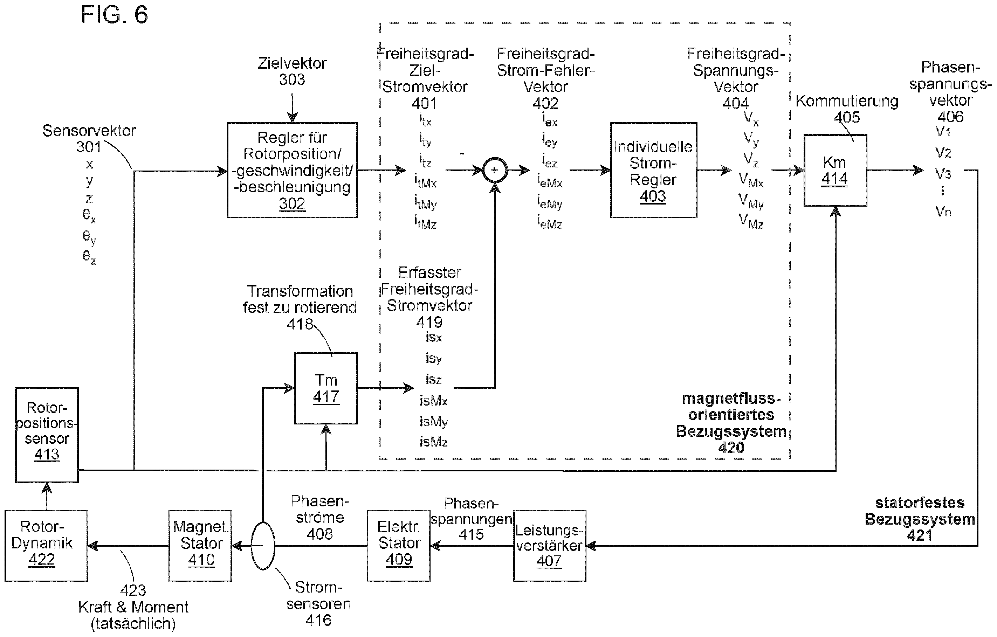

FIG. 6 zeigt ein Regelungsschema, dass ein weiteres Beispiel des angegebenen Steuerverfahrens implementiert. Motivation hierfür ist, wie bereits oben erwähnt, dass bei schnell drehenden Motoren Stromregler in der Endstufe schnell veränderlichen Vorgaben ausgesetzt sind. Dies führt zu einer Verzugszeit und damit Phasenverschiebung zwischen Ist- und Sollstrom. Dieses Problem wurde im Stand der Technik für Synchronmaschinen und Asynchronmaschinen mit der feldorientierten Regelung gelöst.

Dieses Konzept kann auf eine Regelung von 6 Freiheitsgraden in einem n-Phasen-Motor erweitert werden. Dabei umfasst das Bestimmen der Mehrzahl von Steuervorgaben: Bestimmen eines jeweiligen Phasenstroms durch jede der Mehrzahl von Spuleneinheiten und Transformieren der bestimmten Phasenströme 408 in Anteile eines Freiheitsgrad-Stromvektors 419, die einer jeweiligen Verschiebung der aktuierbaren Komponente (Rotor) in Richtung der Achsen eines bezüglich der stationären Komponente ortsfesten Koordinatensystems und/oder einer jeweiligen Drehung der aktuierbaren Komponente um die Achsen dieses Koordinatensystems entsprechen. Diese Transformation definiert ein Koordinatensystem, das hier als magnetflussorientiertes Bezugssystem 420 bezeichnet wird. Das bisher betrachtete (nicht mitrotierende) Koordinatensystem wird hier als statorfestes Bezugssystem 421 bezeichnet.

Die mittels Stromsensoren 416 gemessenen Phasenströme 408 werden somit von einer rotorpositionsabhängigen Matrix 417 in einen virtuellen Stromvektor (erfasster Freiheitsgrad-Stromvektor 419) überführt, wobei jeder Strom der Kraft- oder Momenterzeugung eines Freiheitsgrades entspricht. Die rotorpositionsabhängige Matrix 417 entspricht Tₘ, ggf. spaltenweise oder zeilenweise skaliert, um von Kräften oder Momenten in Ströme umzurechnen.

Jede der 6 Stromkomponenten kann nun im mitrotierenden Bezugssystem 420 einzeln geregelt werden. In anderen Worten erfolgt ein Bestimmen einer jeweiligen transformierten Steuervorgabe für jede der Koordinaten des rotierenden Koordinatensystems 420 unter Verwendung eines jeweiligen Reglers (Individuelle Stromregler 403). Die individuellen Stromregler 403 sind von der Rotorbewegung entkoppelt. Der Grad der Entkopplung hängt davon ab, welche Komponenten der durch den Rotorpositionssensors 413 erfassten Position an Tₘ und Kₘ zurückgeführt werden. Für die übliche feldorientierte Regelung wird nur die Rotation um die Antriebsachse zurückgeführt. Dies kann auch für die Regelung in mehreren Freiheitsgraden hinreichend sein. Allerdings können auch mehr bis alle Freiheitsgrade zurückgeführt werden. Hierdurch können die Stromregler 403 beispielsweise von einer durch Unwucht erzeugten Schwingung entkoppelt werden.

Die individuellen Stromregler 403 geben jeweils eine Spannung für einen Freiheitsgrad aus (zusammengefasst im Freiheitsgrad-Spannungsvektor 404). Diese bis zu sechs Freiheitsgradspannungen werden anschließend, ähnlich der Matrix-Kommutierung, durch Multiplikation mit der rotorpositionsabhängigen Kₘ Matrix in Phasenspannungsvorgaben (Phasenspannungs-Vektor 406 = Steuervorgabenvektor) umgerechnet (Rücktransformieren der transformierten Steuervorgaben zum Bestimmen der Mehrzahl von Steuervorgaben). Kₘ muss hierfür gegebenenfalls zeilenweise skaliert werden. Diese Phasenspannungsvorgaben (Phasenspannungs-Vektor 406) können anschließend von der Endstufe (Leistungs-Verstärker 407) an die Phasen angelegt werden (Phasenspannungen 415). Einzelne Stromregelungen oder Vorsteuerungen in der Endstufe sind meist nicht mehr notwendig.

Für den Fall, dass der Rotorpositionssensor 413 nur die Drehung um die Antriebsachse ausgibt und drei Motorphasen mit zwei Stromfreiheitsgraden existieren, ergeben sich für Tₘ die bekannte Clarke-Park-Transformation und für Kₘ die inverse Park-Clarke-Transformation und somit die bekannte ein- oder zweidimensionale feldorientierte Regelung. Wobei für Radialflussmotoren nur eine eindimensionale Steuerung des Antriebsmoments sowie eine Feldschwächung, welche allerdings ebenfalls nur die Drehung betrifft, möglich ist. Bei einseitigen Axialflussmotoren führt eine Feldschwächung dazu, dass der Stator den Rotor abstößt und damit axiale Kräfte erzeugt. Mit negativer Feldschwächung, also Feldverstärkung, kann der Rotor angezogen werden. Die übliche feldorientierte Regelung ist damit in der Lage, einen einseitigen Axialflussmotor zweidimensional, nämlich in den Dimensionen Mz und Fz, mit Rotationsachse z, zu kontrollieren.

Gyroskopische Effekte, wie beispielsweise Präzession oder Nutation, führen bei größeren Drehzahlen dazu, dass eine Regelung, insbesondere der Ausrichtung der Rotationsachse, mit den (bei geringen Drehzahlen stabilen) Reglern für jeden Freiheitsgrad nicht mehr stabil ist. Die Präzession führt bei einem um die z-Achse drehenden Rotor zu einer Verkippung um die x-Achse, wenn Drehmoment um die y-Achse erzeugt wird. Diese Verkopplung der Freiheitsgrade kann bei der Auslegung der Regler für die einzelnen Freiheitsgrade berücksichtigt werden, insbesondere durch Verkopplungen zwischen den Reglern. Aus dem Stand der Technik sind hierzu entsprechende Methoden bekannt (beispielsweise doi:10.1109/87.531916).

Anknüpfend an die weiter oben erläutere Steuerbarkeitsanalyse, soll nun ein Betrieb im Fehlerfall diskutiert werden. Die Auswirkungen von verschiedenen Fehlerfällen wie beispielsweise Überhitzung, Kabelbruch, elektrischem Durchschlag von Drahtisolation oder von Halbleiterkomponenten, mechanische Beschädigung, mechanische Überlastung oder defekten Lötstellen, lassen sich beispielsweise folgenden Kategorien zuordnen: offene Phase, teilweise oder ganz kurzgeschlossene Phase, ein Kurzschluss zwischen benachbarten Phasen, offen ausgefallener Halbbrückenschalter und geschlossen ausgefallener Halbbrückenschalter.

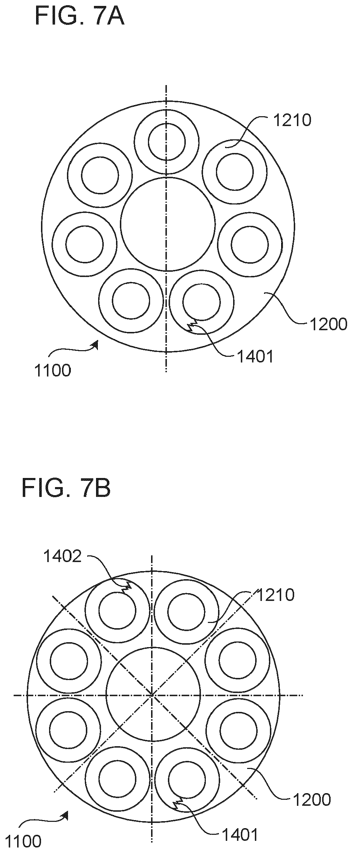

FIG. 7A zeigt exemplarisch die stationäre Komponente 1200 der elektrischen Baugruppe 1100 nach FIG. 1A bis FIG. 1C, wobei hier ein Bruch einer Spuleneinheit 1210 (d. h. offene Phase) als Erstfehler 1401 aufgetreten ist. FIG. 7B zeigt einen achtphasigen Stator, bei dem ein Erstfehler 1401 wie in FIG. 7A und zusätzlich ein Bruch einer weiteren Spuleneinheit 1210 als Zweitfehler 1402 aufgetreten ist.

Um auf einen Fehlerzustand reagieren zu können, muss dieser zunächst erfasst werden. Das Steuerverfahren kann dazu umfassen: Erfassen mindestens einer Messgröße, die ein Erfassen eines Fehlerzustands der elektrischen Baugruppe ermöglicht, wobei der Fehlerzustand insbesondere ein Ausfallen mindestens einer ersten Spuleneinheit der Mehrzahl von Spuleneinheiten und/oder eine nicht vorgesehene elektrische Verbindung mindestens zweier Spuleneinheiten der Mehrzahl von Spuleneinheiten umfasst. Dies kann durch ständige Spannungs- oder Strommessung in den Phasen erfolgen. Werden die Phasen nicht einzeln gemessen, dann kann auch der Summenstrom gemessen werden. Die Zuordnung zu den einzelnen Phasen kann per Beobachter oder ein in jede Phase individuell eingeprägtes und identifizierbares Signal erfolgen.

Ein Vorteil der sieben- oder mehr-phasigen Baugruppe 1100 ist, dass jedenfalls im Erstfehlerfall weiterhin alle Freiheitsgrade geregelt werden können, wenn der Betrieb entsprechend angepasst wird (in einer Situation, in der dies nicht mehr gegeben ist, kann beispielsweise eine Warnung ausgegeben und/oder der Betrieb beendet werden).

Die Steuerbarkeit kann wie weiter oben beschrieben analysiert werden. Die Steuerbarkeit mit einer ausgefallen Phase ist in FIG. 8 für verschiedene Anzahlen von Phasen und Rotor-Polpaaren sowie einer ursprünglich symmetrischen Anordnung der Spulen dargestellt. Für diesen Fall ist der siebenphasige Motor mit 2, 5, und 9 Polpaaren auch im Fehlerzustand noch voll steuerbar. Die Steuerbarkeit ist in diesem Fall nur für Motoren mit 5 oder weniger unabhängigen Phasen durch den Fehlerzustand degradiert.

In den oben genannten Fehlerfällen, mit Ausnahme des geschlossen ausgefallenen Halbbrückenschalters, ist es möglich, die betroffene Phase stromlos zu schalten. Für den Fall eines Schlusses zwischen zwei Phasen ist es ausreichend eine der betroffenen Phasen zu deaktivieren. Der siebenphasige Axialflussmotor verhält sich nach Deaktivierung einer Phase wie ein sechsphasiger Axialflussmotor mit unsymmetrischer oder lückenhafter Verteilung der Spulen (FIG. 7A).

Die Asymmetrie sorgt dafür, dass mit den verbleibenden sechs Phasenströmen trotzdem alle Freiheitsgrade geregelt werden können. Dabei müssen alle Komponenten der Kₘ-Matrix neu bestimmt werden. Bei einem Erfassen des Fehlerzustands ist also insbesondere vorgesehen: Anpassen einer Vorschrift zum Bestimmen der Mehrzahl von Steuervorgaben derart, dass das Steuern und/oder Regeln der Position und/oder Bewegung der aktuierbaren Komponente relativ zu der stationären Komponente unter Verwendung der Mehrzahl von Spuleneinheiten unter Berücksichtigung des Fehlerzustands, insbesondere ohne die erste Spuleneinheit, erfolgt.

In dem vorliegenden Spezialfall mit 6 verbleibenden Spulen und 6 Freiheitsgraden ist Tₘ quadratisch, sodass Kₘ direkt mit der normalen Matrixinversion berechnet werden kann. Eine weitere Optimierung, beispielsweise der Verlustleistung, ist nicht mehr möglich, da keine Dimension zur Verfügung steht und die Matrixinversion eindeutig ist. Es ergeben sich 7 verschiedene Kₘ-Matrizen, je eine für die Deaktivierung einer definierten Phase.

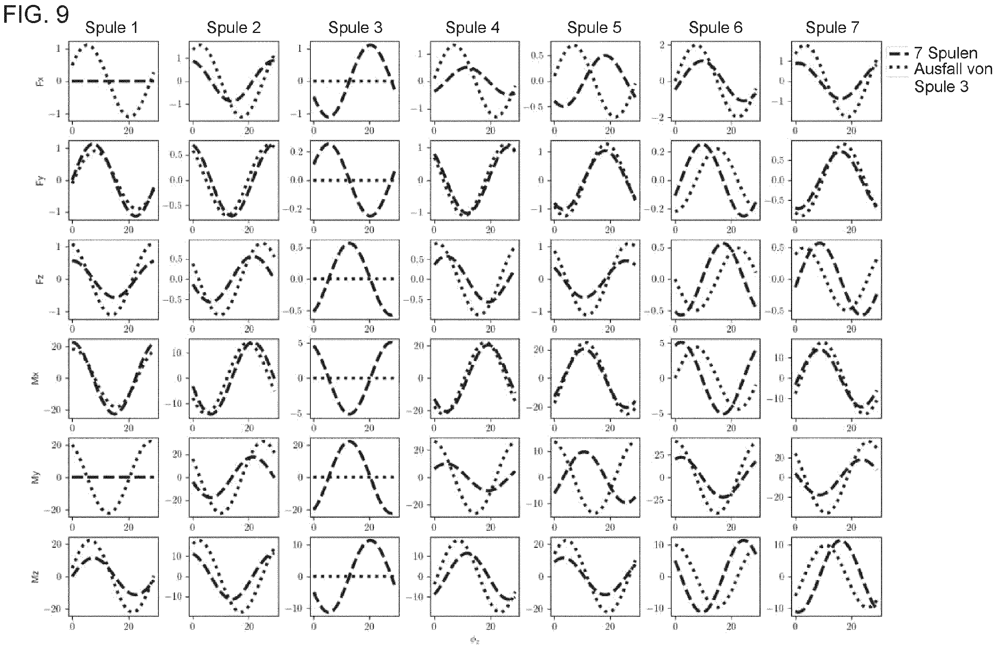

Beispielhaft veranschaulicht FIG. 9 den Unterschied zwischen Kₘ im Normalfall (7 Spulen) und Kₘ₃ für einen Ausfall von Phase 3. Der Notfallbetrieb besitzt einen verringerten Wirkungsgrad bei jedoch weiterhin voller Rotorsteuerbarkeit. Die Einheiten entsprechen den Einheiten aus Fig. 5 mit N/A für die Kräfte und Nm/A für die Drehmomente. Die Abszissen sind hier mit der mechanischen Rotorverdrehung um die z-Achse beschriftet. Die Veränderung der Einträge der Kommutierungsmatrix Km ist nur beispielhaft über der Rotation um die z-Achse aufgetragen. Es kann vorteilhaft sein, Km ebenso mit der Position der aktuierbaren Komponente in weiteren Freiheitsgraden, insbesondere allen Freiheitsgraden, zu variieren.

Bei mehr als sieben Phasen können theoretisch auch Zweitfehler (wie in FIG. 7B gezeigt) toleriert werden. Im besonderen Fall von acht Phasen können Zweifehler allerdings dazu führen, dass eine symmetrischer 6-Phasen Motor resultiert, welcher unter Umständen nur in 5 Freiheitsgraden vollständig quasistatisch steuerbar ist (FIG. 7B). Daher ist der symmetrische achtphasige Motor zwar statistisch ausfallsicherer, allerdings nicht generell zweitfehlersicher.

Der neunphasige Axialflussmotor ist damit der kleinste symmetrische zweitfehlersichere Axialflussmotor mit Steuerbarkeit in sechs Freiheitsgraden. Vorteilhaft ist weiterhin, dass dreiphasige Motoren mit drei oder sechs Spulen pro Phase bereits in Serie gefertigt werden. Diese können ohne Änderung der Wicklung, lediglich durch eine andere Verschaltung der Einzelspulen, neunphasig betrieben werden.

Mit mehr als neun Phasen kann die Ausfallsicherheit weiter gesteigert werden. Allerdings wird der Mehrwert immer geringer, da weiterhin einzelne Komponenten wie die Steuereinheit anfällig für Erstfehler sein können. Für Anwendungen mit weiter erhöhter Ausfallsicherheit kann es dann u. U. sinnvoll sein, zwei separate erstfehlersichere Statoren an denselben Rotor anzukoppeln. Hierfür kann in weiteren Ausgestaltungen auch die selbe Magnetanordnung verwendet werden, so dass der Rotor nicht größer oder schwerer werden muss.

Ist der Fehler identifiziert, dann können die entsprechenden Endstufen abgeschaltet werden und die Kommutierungsmatrix Kₘ, und gegebenenfalls auch Tₘ, entsprechend angepasst werden. Hierzu können grundsätzlich mehrere Matrizen vorgehalten werden. Werden die Matrixeinträge zur Laufzeit berechnet, dann kann die Rechenvorschrift geändert werden. Für das obige Beispiel einer Umschaltung von 7 auf 6 Phasen reicht die Anpassung von Amplitude und Phase für die Generatorfunktion jedes Eintrags der Kₘ-Matrix.

Hierdurch ist es möglich, unterbrechungsfrei auf den Fehler zu reagieren. Durch die Regelung aller Freiheitsgrade stehen zudem umfassende Diagnosemöglichkeiten zur Verfügung, um den Schaden sowie dessen Auswirkungen abzuschätzen. Darauf basierend können Reparaturen vorbereitet werden und die Ausfallzeit minimiert werden.

Hat die elektrische Baugruppe weniger Phasen als zu regelnde Freiheitsgrade, (oder eine degradierte Steuerbarkeit) dann ist eine quasistatische Steuerung oder Regelung nicht möglich. Tₘ hat dann die Dimension f x p (f: Anzahl Freiheitsgrade, p: Anzahl unabhängiger Phasen): $\left(\begin{matrix}{F}_{x} \\ {F}_{y} \\ {F}_{z} \\ {M}_{x} \\ {M}_{y} \\ {M}_{z}\end{matrix}\right) = FM = {T}_{m} ⋅ {I}_{p} = \left[\begin{matrix}{c}_{Fxi 1} & {c}_{Fxi 2} & … & {c}_{Fxip} \\ {c}_{Fyi 1} & {c}_{Fyi 2} & … & {c}_{Fyip} \\ {c}_{Fzi 1} & {c}_{Fzi 2} & … & {c}_{Fzip} \\ {c}_{Mxi 1} & {c}_{Mxi 2} & … & {c}_{Mxip} \\ {c}_{Myi 1} & {c}_{Myi 2} & … & {c}_{Myip} \\ {c}_{Mzi 1} & {c}_{Mzi 2} & … & {c}_{Mzip}\end{matrix}\right] ⋅ \left(\begin{matrix}{i}_{1} \\ {i}_{2} \\ … \\ {i}_{p}\end{matrix}\right) .$

Für p < 6 sind nicht alle Komponenten von FM unabhängig steuerbar. Das heißt nicht zwangsläufig, dass einige Komponenten von KM nicht steuerbar sind, sondern kann auch heißen, dass die Freiheitsgrade verkoppelt sind.

Deswegen ist eine quasistatische Regelung in f Freiheitsgraden nicht möglich. Es kann jedoch eine dynamische Regelung (wie oben definiert) möglich sein. Bei dem vorgenannten Steuerverfahren kann beispielsweise vorgesehen sein, dass nach Anpassen der Vorschrift durch Ansteuern einer beliebigen Kombination von höchstens fünf der Mehrzahl von Spuleneinheiten eine Position und/oder Bewegung der aktuierbaren Komponente relativ zu der stationären Komponente unter Ausnutzung einer Trägheit der aktuierbaren Komponente in einem bewegten, insbesondere rotierenden, Zustand der aktuierbaren Komponente in den sechs linear unabhängigen Freiheitsgraden steuerbar ist. Es kann also insbesondere von einer quasistatischen Ansteuerung im Normalbetrieb zu einer dynamischen Ansteuerung bei Erfassen des Fehlerzustands gewechselt werden.

Dies soll noch etwas näher ausgeführt werden. Für den Fall, dass der Rotor sich bewegt, beispielsweise dreht, verändert sich die Steuerbarkeit über die Bewegung. Um hierdurch eine Regelung der gewünschten f Freiheitsgrade zu ermöglichen, müssen zwei Bedingungen erfüllt sein:
1. Die Verkopplungen (Spalten der verschiedenen Tₘ-Matrizen) bei verschiedenen Rotorpositionen müssen insgesamt einen Vektorraum aufspannen, der f-dimensional ist.
2. Die Bewegung des Rotors muss schnell genug sein, so dass die Trägheit des Rotors die Rotorkräfte und -momente mittelt.

Um die möglichen Korrekturkräfte zu ermitteln kann dann wie folgt vorgegangen werden:
1. Rotorposition bestimmen,
2. gewünschten Korrektur-Kraft-Moment-Vektor ermitteln (unter Berücksichtigung von Trägheit, passiven Lagerkräften, Lastkräften oder gyroskopischen Momenten),
3. Ermittlung von Tₘ für die aktuelle Position,
4. Bestimmung des möglichen Vektorraums, der von den Spalten von Tₘ aufgespannt wird,
5. Ermittlung eines somit möglichen Korrektur-Kraft-Moment-Vektors, welcher möglichst nah an dem gewünschten Korrektur-Kraft-Moment-Vektor liegt,
6. Einprägen der Ströme für diesen möglichen Korrektur-Kraft-Moment-Vektor.

Ebenso denkbar ist es, die im Zusammenhang mit FIG. 4 und FIG. 6 erläuterten Verfahren zu modifizieren und einzelne, bei der aktuellen Rotorposition schlecht steuerbare Freiheitsgrade abzuschwächen oder abzuschalten.

Alternativ können modellbasierte Verfahren wie beispielsweise Model Predictive Control und/oder eine Zustandsrückführung eingesetzt werden.

Die Regelung kann mit den Verfahren so eingeschränkt sein, dass eine Positionsregelung in bestimmten Freiheitsgraden oder Linearkombination von Freiheitsgraden nicht mehr möglich ist. In diesen Fällen kann die Regelung aber häufig noch zum Dämpfen eingesetzt werden. Hierbei können passive Verkopplungen zwischen Freiheitsgraden, beispielsweise durch gyroskopische Effekte oder durch passive Magnetlager, hilfreich sein, da die Dämpfung eines Freiheitsgrads auch alle passiv verkoppelten Freiheitsgrade bedämpft.

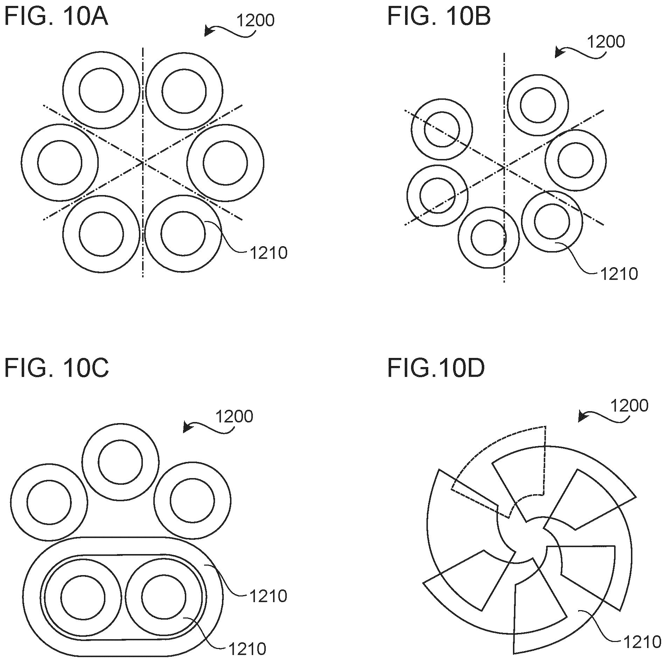

Oben wurde bereits erwähnt, dass die Steuerbarkeit durch eine Symmetrie des Stators degradiert sein kann, wie sie etwa bei einem sechsphasigen Stator mit äquidistant um die Rotationsachse herum angeordneten Spuleneinheiten 1210 gegeben ist (FIG. 10A). Im Fall des Fehlerbetriebs wurde auch bereits darauf eingegangen, wie dieser Degradierung durch eine asymmetrische Anordnung entgegengewirkt werden kann. Diese Wirkung kann auch im Normalbetrieb ausgenutzt bzw. bei der Ausgestaltung der elektrischen Baugruppe berücksichtigt werden.

Bei den Beispielen, die in FIG. 10B bis FIG. 10D gezeigt sind, ist vorgesehen, dass jeweilige magnetische Komponenten mindestens zweier Spuleneinheiten 1210 der Mehrzahl von Spuleneinheiten 1210 zueinander asymmetrisch, insbesondere nicht äquidistant, angeordnet und/oder unterschiedlich dimensioniert und/oder unterschiedlich geformt sind.

So kann der sechsphasige Stator durch eine Lücke zwischen zwei Spulen (FIG. 10B) die volle Steuerbarkeit erlangen. Diese Anordnung entspricht einem siebenphasigen Stator mit ausgefallener Phase. Eine beispielhafte alternative Anordnung eines sechsphasigen Stators umfasst jeweils eine Lücke zwischen Gruppen von jeweils zwei Spulen (entsprechend einem neunphasigen Stator mir drei ausgefallenen Phasen in gleichen Abständen).

Zur besseren Ausnutzung der verfügbaren Fläche kann (alternativ oder zusätzlich zu den genannten Asymmetrien/Lücken in der Anordnung) die Form der Spulen voneinander abweichen (FIG. 10C, FIG. 10D).

Die Asymmetrie kann zusätzlich oder alternativ oder zusätzlich zu der Anordnung oder Beschaffenheit der Spulen auch mit unterschiedlich geformten oder unsymmetrisch verteilten Statorzähnen erzeugt werden um die volle Steuerbarkeit zu erreichen.

Im Folgenden wird noch auf spezielle Aspekte bzw. Ausgestaltungen des Steuerverfahrens eingegangen.

Liegt die eingeregelte Rotationsachse nicht auf dem Massenschwerpunkt des Rotors, dann erzeugt die Antriebsrotation eine Unwucht, welche Kräfte auf den Rotor und Gegenkräfte auf den Stator überträgt. Nicht nur der Massenschwerpunkt spielt hierbei eine Rolle, sondern auch die Lager der Rotationsachse relativ zu den Hauptträgheitsachsen des Rotors.

Um den negativen Auswirkungen solcher Unwuchten (Vibration, Beanspruchung) zu begegnen, kann das Steuerverfahren umfassen: Minimieren einer Unwucht der aktuierbaren Komponente 1300 bezüglich einer Rotation relativ zu der stationären Komponente 1200 um eine Rotationsachse 1400 durch dynamisches Festlegen und/oder Anpassen einer Position und/oder Orientierung der Rotationsachse 1400 in Bezug auf die aktuierbare Komponente 1300.

Verfahren für eine solche automatische Minimierung der Unwucht durch dynamische Wahl der Rotationsachse relativ zum Rotor sind grundsätzlich bekannt.

Dies gilt sowohl für eine Unwuchtsminimierung in einer Ebene, als auch in bis zu sechs Freiheitsgraden.

Ist nicht die Bewegung des Rotors, sondern die Schwingung des Stators zu minimieren, dann kann diese mit mindestens einem am Stator angebrachten Beschleunigungssensor und/oder Kraftsensor ermittelt werden und die Rotationsachse entsprechend angepasst werden. Für Unwuchten oder Schwingungen höherer Ordnung ist eine rein statische Positionierung der Rotationsachse nicht ausreichend. Für diese Fälle können kontinuierlich Kompensationskräfte ermittelt und eingeprägt werden.

Da alle Rotormagnete für die Generierung von Antriebsmoment und die Lagerung zur Verfügung stehen, kann der Rotor bei den vorgestellten Baugruppen besonders leicht sein. Dies führt zu geringen Unwuchtskräften und in Kombination mit der Methode des Ausregeln von Unwuchten zu besonders kleinen Unwuchtskräften.

Die Kₘ-Matrix wurde in den vorangegangenen Beispielen theoretisch bestimmt. Dabei werden bestimmte Annahmen über beispielsweise das Magnetfeld des Rotors, die Spulengeometrie, die Eisengeometrie und die Symmetrie aller Komponenten getroffen. In der Realität wird es immer einen Unterschied zwischen diesen Annahmen und dem tatsächlichen Aufbau geben. Handelt es sich dabei um systematische Abweichungen, dann können diese für die vorliegende Maschinenserie an beispielhaften Mustern vermessen werden. Dies deckt allerdings Prozessschwankungen oder Alterung nicht ab.

Durch die Messung und Regelung der Rotorposition in idealerweise sechs Freiheitsgraden, kann sich die elektrische Baugruppe selbst vermessen. Mit weniger Freiheitsgraden sinkt der Informationsgehalt, eine äquivalente Vermessung ist aber ebenso möglich.

Es kann also vorgesehen sein, dass die Kommutierungsmatrix unter Verwendung einer Messung bestimmt und/oder korrigiert wird. Eine solche Messung kann beispielsweise mittels Kraft- und/oder Momentsensoren an der aktuierbaren Komponente 1300 erfolgen. Eine Messung kann auch durch Bewegung der aktuierbaren Komponente 1300 in verschiedenen Richtungen, insbesondere den linear unabhängigen Freiheitsgraden, und Erfassen einer durch diese Bewegungen in den Spuleneinheiten 1210 induzierten elektromotorischen Gegenkraft (Back-EMF) erfolgen. Diese Möglichkeiten werden im Folgenden noch näher erläutert.

Eine weitere vorgeschlagene Methode zur Selbstvermessung und Korrektur der Kₘ-Matrix setzt voraus, dass die initiale Kₘ-Matrix bereits in der Lage ist, den Rotor quasistatisch in den zu regelnden Freiheitsgraden auszuregeln. In diesem Zustand werden dann Signale auf einzelne Kraft- oder Momentkomponenten aufgeprägt. Diese Signale und die daraus resultierenden Kräfte und Momente führen dazu, dass der Rotor sich bewegt. Diese Bewegung wird mit den Freiheitsgraden des Rotorpositionssensors, oder mit den Reaktionen der verbleibenden Regler, bestimmt. Die gemessenen Sensorsignale werden anschließend auf Signalkomponenten welche zu den aufgeprägten Signalen korrelieren, untersucht. Die Korrelation sollte in dem angepeilten Freiheitsgrad am größten sein, da die Regelung sonst nicht stabil wäre. Die Korrelation in den Freiheitsgraden, welche nicht zu dem speziellen Anregungssignal gehören, sind Indikatoren für eine Querabhängigkeit. Solch eine Querabhängigkeit kann beispielsweise entstehen, wenn die Sensorausrichtung, und damit das Sensorkoordinatensystem nicht mit dem Statorkoordinatensystem übereinstimmt.

Ist die Sensorbaugruppe beispielsweise um 10° um die z-Achse verdreht, dann vermischen sich scheinbar x, und y-Krafterzeugung. Sollte Kraft in x-Richtung erzeugt werden, dann ist die tatsächliche x-Kraft auf cos(10°)=98,5% reduziert. Gleichzeitig wird aber eine unbeabsichtigte y-Kraft mit sin(10°)=17,4% der beabsichtigten x-Kraft erzeugt. Die Korrelation ermittelt dann genau diese 17,4% Quereinfluss. Aus der vorläufigen Kₘ-Matrix ist bekannt, welche Ströme eigentlich diese 17,4% y-Kraft erzeugen sollten. Diese Stromkomponenten werden anschließend von den Kₘ Einträgen für die x-Kraft abgezogen.

Iteriert man dieses Verfahren für alle Freiheitsgrade, bei Bedarf mehrfach, dann passt sich Kₘ an die tatsächlichen Gegebenheiten der Baugruppe an. Die Methode vermisst effektiv die Sensorcharakteristik mit Bezug auf die Charakteristik der Aktoren.

Die Anpassung kann für mehrere elektrische oder auch mechanische Rotorwinkel sowie Verschiebungen in den anderen Freiheitsgraden wiederholt werden. Hierdurch kann Km an die individuelle Feldform des Rotors sowie die Form der Spulen und Eisenkomponenten angepasst werden.

Die Abschätzung für eine Sensorverdrehung von 10° verdeutlicht, dass das System empfindlich auf Querabhängigkeiten reagiert. Die Verkopplung der Regler aller Freiheitsgrade erhöht die Störung in jedem Regler weiter. Entsprechende Kompensationsmethoden sind daher nicht als weitere Entwicklung, sondern für Aufbauten mit bestimmten Toleranzen, oder Vereinfachungen bei der Auslegung, als eine Voraussetzung für den sicheren Betrieb anzusehen.

Die aufgeprägten Signale müssen lediglich in den Sensorsignalen identifizierbar sein. Hierfür kommen beispielsweise Sinusschwingungen, aber auch Pseudozufallssignale (PRBS) oder ein einfacher Offset, in Betracht. Sind die Signale zudem voneinander unterscheidbar, dann können mehrere Freiheitsgrade gleichzeitig korrigiert werden. Auch während des normalen Betriebs der Baugruppe können die Signale in ausreichend kleiner Amplitude aufgeprägt werden, um Alterung oder Drift zu verfolgen oder auch zu korrigieren.

Die Vermessung kann auch erfolgen, wenn der Rotor passiv gehalten wird. Hierfür kann die elektrische Baugruppe eine Stützvorrichtung zum Stützen der aktuierbaren Komponente in einem Freiheitsgrad derart umfassen, dass die aktuierbare Komponente in diesem Freiheitsgrad relativ zu der stationären Komponente passiv stabil gelagert ist. In diesen Aufbau ist eine Kleinsignalanalyse der Querabhängigkeiten der Kommutierungsmatrix möglich.

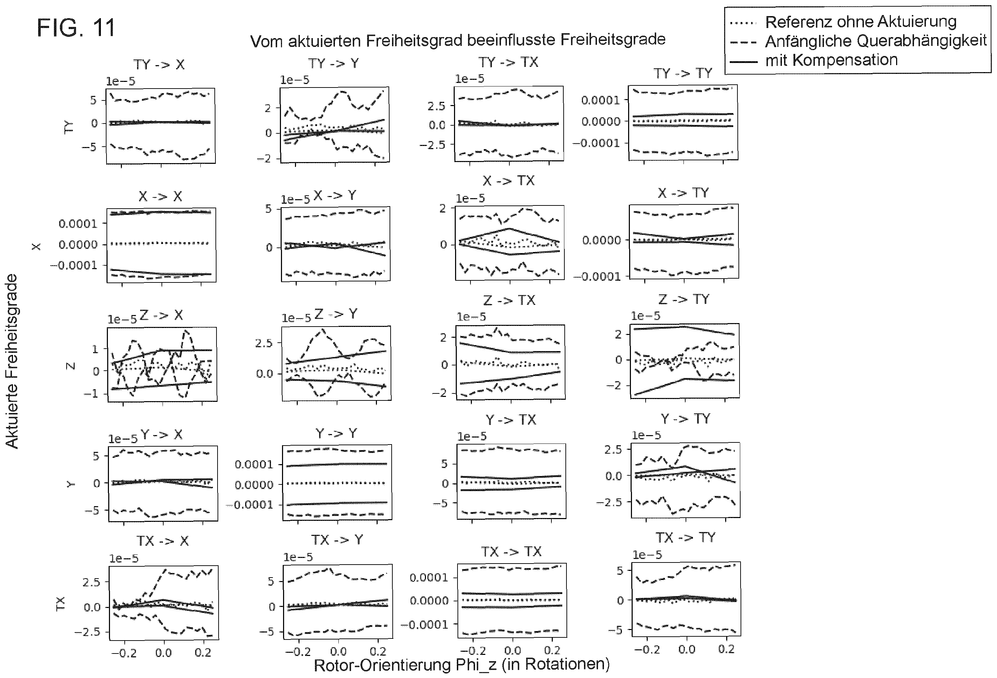

FIG. 11 zeigt das Ergebnis einer beispielhaften Messung. Der Rotor ist in z-Richtung an einem Faden aufgehängt. Es werden daher nur Fx, Fy, Mx und My vermessen. Durch optionale Gegenregelung um die z-Achse und Kraftmessung am Faden können auch Fz und Mz bestimmt und korrigiert werden, dies ist im Beispiel nicht erfolgt.

Jeder Plot in FIG. 11 zeigt den Verlauf der Rotorposition in einem bestimmten Freiheitsgrad bei einer Aktoransteuerung (aktuierte Freiheitsgrade) in einem weiteren bestimmten Freiheitsgrad. Es wurde dabei einmal in positive und einmal in negative Richtung ausgesteuert, um Offsets unterdrücken zu können. Jeder Plot ist mit {"Aufgeprägte Kraft oder Moment" → "Sensorkanal"} beschriftet. In den Plots, wo Aktor-Freiheitsgrad und Sensor-Freiheitsgrad übereinstimmen, sind die Auswirkungen erwartungsgemäß groß und relativ unabhängig vom Kommutierungswinkel.

Km kann nach der Messung folgendermaßen korrigiert werden:
Aus einem Plot (wie in FIG. 11) Koeffizienten von x_a, y_a, tx_a und ty_a bestimmen oder ablesen, sodass: $x_a \left\{x, y, tx, ty\right\} = Sensorwerte \left(x, y, tx, ty\right) / A_x$ mit Ax der Amplitude der Anregung im Experiment.

Aufstellen des Gleichungssystems für Korrektur des Aktors Fx: $\begin{matrix}\left(\begin{matrix}{S}_{x = 1} \\ {S}_{y = 0} \\ {S}_{tx = 0} \\ {S}_{ty = 0}\end{matrix}\right) = \left[\begin{matrix}{x}_{ax} & {y}_{ax} & {tx}_{ax} & {ty}_{ax} \\ {x}_{ay} & {y}_{ay} & {tx}_{ay} & {ty}_{ay} \\ {x}_{atx} & {y}_{atx} & {tx}_{atx} & {ty}_{atx} \\ {x}_{aty} & {y}_{aty} & {tx}_{aty} & {ty}_{aty}\end{matrix}\right] ⋅ \left(\begin{matrix}a \\ b \\ c \\ d\end{matrix}\right) ⇔ {S}_{x 0} = CS ⋅ \left(\begin{matrix}a \\ b \\ c \\ d\end{matrix}\right) \\ ⇔ {CS}^{- 1} ⋅ {S}_{x 0} = \left(\begin{matrix}a \\ b \\ c \\ d\end{matrix}\right)\end{matrix}$ mit CS - CrossSensitivity Matrix und Matrixeinträgen [actor-dof]_{a[affected dof]}.

Der korrigierte Kₘ-Matrixeintrag Kₘ₂ für die Achse X ist dann ${K}_{m 2} = {K}_{mx} ⋅ a + {K}_{my} ⋅ b + {K}_{mtx} ⋅ c + {K}_{mty} ⋅ d$ mit K_{m{DOF}}: Stromvektor für die Erzeugung von Kraft im {DOF=Freiheitsgrad}.

Die Berechnung kann für die weiteren Aktor-Freiheitsgrade mit anderen a....d wiederholt werden.

Alternativ kann die Kommutierungsmatrix mit kompensierten Querabhängigkeiten K_{m_comp} direkt berechnet werden: ${K}_{m _ comp} = {CS}^{- 1} ⋅ {K}_{m} .$

Entsprechend der vorangehenden Beschreibung umfasst also das Steuerverfahren: Bestimmen einer Querabhängigkeit und Bestimmen und/oder Anpassen der Steuervorgaben unter Berücksichtigung der bestimmten Querabhängigkeit zum Minimieren der Querabhängigkeit.

In den bisherigen Beispielen war die aktuierbare Komponente/der Rotor auf einer Seite der stationären Komponente/des Stators angeordnet. Die elektrische Baugruppe kann jedoch eine zweite aktuierbare Komponente/einen zweiten Rotor umfassen. Die zweite aktuierbare Komponente kann mittels der Mehrzahl von Spuleneinheiten der stationären Komponente in gleicher Weise steuerbar sein wie die vorgenannte aktuierbare Komponente. Aus dem Stand der Technik sind grundsätzlich Axialflussmotoren mit beidseitigem Rotor zur Steigerung der Effizienz bekannt.

Stellt man beispielsweise für einen beidseitigen dreiphasigen Axialflussmotor Tₘ auf, dann ist daraus erkennbar, dass das Antriebsmoment Mz effizienter erzeugt werden kann. Die Erzeugung von Kraft F_{z} ist bei den beiden Rotorhälften allerdings entgegengerichtet und kann sich sogar komplett aufheben.

Wenn die Generierung von F_{z} weiterhin erwünscht ist, allerdings weniger Effizienz notwendig ist, dann kann eine Rotorhälfte mit kleineren oder schwächeren Magneten ausgestattet werden, um die Kopplung zwischen Spule und Magnet zu reduzieren. Die Antriebsmomenterzeugung profitiert dann von beiden Rotorenhälften, die Krafterzeugung hingegen verhält sich so, als gäbe es nur einen Rotor mit der Differenz an Magneten.

Veranschaulicht verstärkt die zweite Rotorhälfte den axialen Magnetfluss, verringert jedoch den tangentialen Magnetfluss. Die radialen Wicklungsstränge erzeugen Lorentzkräfte senkrecht zum Feld. Ohne tangentiale Magnetflussanteile kann keine Kraftwirkung in axialer Richtung erzeugt werden.

Erweitert auf den sechs- oder mehr -phasigen Motor zeigt sich, dass jede Einzelspule mehr Tangentialkräfte aber weniger Axialkräfte erzeugen kann. Durch die Anordnung der Spulen und Kombination der Kräfte auf den Rotor, sind Mz, Fx und Fy beim asymmetrischen Rotor verstärkt, dafür aber Fz, Mx und My abgeschwächt.

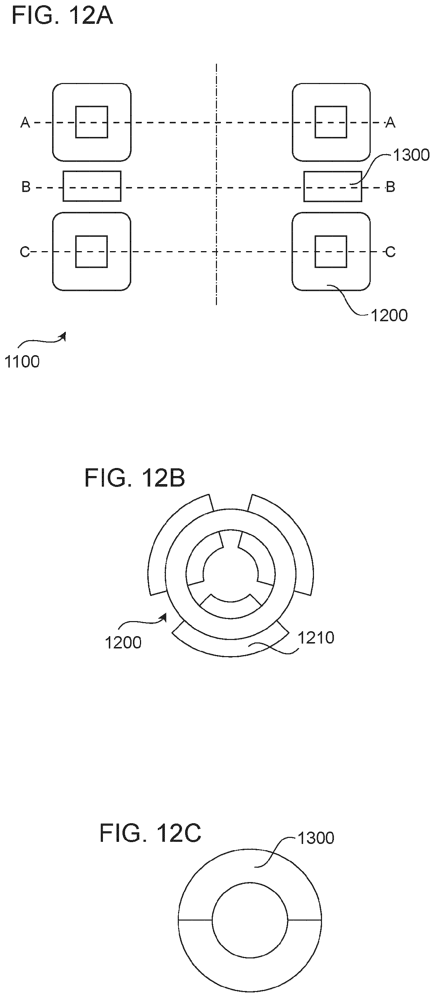

Wie in FIG. 12A bis FIG. 12C gezeigt ist, kann auch vorgesehen sein, dass die Mehrzahl von Spuleneinheiten 1210 eine erste Gruppe von Spuleneinheiten 1210 und eine zweite Gruppe von Spuleneinheiten 1210 umfasst, wobei die Spuleneinheiten 1210 der ersten Gruppe zum Bilden eines ersten Axialflussmotor-Stators in einer ersten Ebene A--A angeordnet sind und die Spuleneinheiten 1210 der zweiten Gruppe zum Bilden eines zweiten Axialflussmotor-Stators in einer zweiten (zu der ersten Ebene A--A parallelen) Ebene C--C angeordnet sind.

Fig. 12B zeigt den Schnitt A--A der ersten Gruppe von Spuleneinheiten. Die zweite Gruppe von Spuleneinheiten kann ähnlich der ersten Gruppe aufgebaut sein und zusätzlich zu dieser verdreht positioniert sein.

Fig. 12C zeigt einen Schnitt in der Ebene B--B durch die aktuierbare Komponente 1300 mit einer Magnetanordnung mit einem Polpaar.

Die diskutierte Analyse der Steuerbarkeit der Axialflussmotoren kann auch genutzt werden, um solche Kombinationen von Axialflussmotoren zu evaluieren. Schon die Kombination zweier Axialflussmotoren mit drei individuellen Phasen kann zu einer Steuerbarkeit von sechs Freiheitsgraden mit nur sechs Spulen führen. Ein Vorteil bei der Verwendung von zwei sich gegenüberliegenden Axialflussmotoren ist, dass die statische Anziehungskraft zwischen Rotor und Statoreisen ausgeglichen werden kann, da die beiden Statoreisen in unterschiedliche Richtungen ziehen. Die Statoreisen wiederum erhöhen im Gegensatz zu Luftspulen den Wirkungsgrad.

Um auch hier Redundanz zu erreichen kann einer oder beide Statoren mit vier oder mehr unabhängigen Phasen ausgeführt werden. Der Rotor kann Magnete für jeden Stator enthalten oder dieselben Magnete für beide Statoren verwenden. Letzterer Fall ist in FIG. 12A bis FIG. 12C illustriert.

Bei der Steuerbarkeitsanalyse ergeben sich neben der Anzahl der Spulen im oberen und unteren Stator sowie der Anzahl der Polpaare weitere Konfigurationsparameter. So können beispielsweise die Statoren zusätzlich gegeneinander verdreht werden, was, wie in der rechten Hälfte von Fig. 13 gezeigt, die Steuerbarkeit positiv beeinflussen kann. Der Rotor kann als eine Scheibe mit Magneten ausgeführt sein, bei der beide Seiten je einem Stator gegenüber liegen. Alternativ kann jeder Stator aber auch eine eigene Rotormagnetscheibe aktuieren. In diesem Fall kann auch die Polpaarzahl der beiden Rotormagnetscheiben voneinander abweichen oder die Polposition gegeneinander verdreht sein. Der für die Steuerbarkeit positive Effekt der Verdrehung der Polpositionen bei zwei Rotormagnetscheiben kann bei einer Rotormagnetscheibe auch durch eine schiefe Magnetisierung oder eine stufenförmige Magnetanordnung erreicht werden.

Entscheidend ist, dass die Wirkrichtung der einzelnen Aktorkräfte des ersten und zweiten Stators unterschiedlich, insbesondere über die Antriebsrotationsrichtung verschoben, sind (entsprechend einem Phasenversatz in der Ansteuerung). Diese unterschiedliche Wirkrichtung kann mit den Maßnahmen wie Statorverdrehung oder schiefwinklige Magnetisierung erreicht werden, ist jedoch nicht auf diese begrenzt.

Für den doppelten Axialflussmotor mit einer Scheibe und einer optionalen Verdrehung der Statoren gegeneinander ist die Anzahl der unabhängig steuerbaren Freiheitsgrade in FIG. 13 dargestellt. Links neben der Ordinate ist die Konfiguration eines ersten Stators (2001) und eines zweiten Stators (2002) dargestellt. Die Abszisse zeigt primär die Anzahl der Polpaare des Rotors, ist allerdings zusätzlich in zwei Gruppen mit (2004) und ohne Statorverdrehung (2003) dargestellt.

Die Verdrehung der Statoren ist notwendig, um mit der Konfiguration von 3+3 Spulen eine volle Steuerbarkeit zu erreichen. Dabei ergeben sich für verschiedene Verdrehwinkel verschiedene Grade der Steuerbarkeit. Für einen Verdrehwinkel von 1/3 Spulenabstand sind auch einige der 3+4-Konfigurationen voll steuerbar.

Die vorteilhafte Konfiguration mit 3+3 Spulen und einem Polpaar entspricht dem in Fig. 12A, 12B und 12C dargestellten Ausführungsbeispiel.

Für einige Konfigurationen mit insgesamt 7 oder mehr unabhängigen Phasen kann die volle Steuerbarkeit auch im Fehlerfall gegeben sein. Die Bestimmung der Kommutierungsvorschrift für die verschiedenen Fehlerfälle kann analog mit denselben Methoden erfolgen, mit denen die Kommutierungsvorschrift für den einseitigen Axialflussstator hergeleitet werden kann.

Die Konfiguration mit 6+6=12 unabhängigen Statorphasen ist aus dem Stand der Technik (JP2010279230A) für eine Steuerbarkeit in sechs Freiheitsgraden bekannt, daher sind in Fig. 13 nur demgegenüber neue Konfigurationen mit einer geringeren Anzahl von unabhängigen Statorphasen dargestellt.

Die statische Verdrehung ermöglicht auch mit vier, fünf oder sechs Spulen eine quasistatische Steuerbarkeit in ebenso vielen Freiheitsgraden, was dem theoretischen Limit entspricht, wie Fig. 13 beispielsweise für eine beiden Statoren gemeinsame Magnetanordnung mit einem Polpaar zeigt.

Die für FIG. 13 angenommenen Statoren umfassen nur eine Spule pro Phase. Um die Drehmomentdichte eines Motors zu erhöhen, werden oftmals mehrere Spulen pro Statorphase, auch in Verbindung mit einer Erhöhung der Rotor-Polpaarzahl, eingesetzt. Die Vielzahl an Spulen pro Phase ist als konzentrierte oder verteilte Spulen über den Umfang angeordnet. Die dadurch entstehende Symmetrie reduziert allerdings den Wirkungsgrad oder sogar die Steuerbarkeit in Fx, Fy, Mx und My. Oftmals wird die Mz-Drehmomentdichte und der Mz-Wirkungsgrad stärker gewichtet. Hierfür kann der doppelseitige Axialflussmotor auf der einen Seite mit mehreren Spulen pro Phase und auf der anderen Seite mit einer Spule pro Phase ausgeführt werden. Hierdurch wird durch den einen Stator eine gute Mz-Effizienz sichergestellt und durch den anderen Stator eine gute Steuerbarkeit sichergestellt.

Bei Motoren für geringere Drehzahlen oder höhere Antriebsmomente bestehen die unabhängigen Spuleneinheiten in der Regel aus mehreren voneinander Abhängigen, beispielsweise in Reihe oder parallel geschalteten, Spulen. Die Steuerbarkeit kann besonders eingeschränkt sein, wenn die abhängigen Spulen gleichmäßig über den Umfang verteilt sind. So enthalten die meisten dreiphasigen, in der Regel mit zwei unabhängigen Spuleneinheiten ausgestatteten, bürstenlosen Gleichstrommotoren mehrere konzentrierte oder verteilte Spulen, welche gleichmäßig über den Umfang verteilt positioniert sind.. Um dennoch eine ausreichende Steuerbarkeit in mehreren Freiheitsgraden zu gewährleisten, können die Spulen einer unabhängigen Spuleneinheit nur über einen Teil des Umfangs, insbesondere einen Sektor, insbesondere einen Sektor, der einen Winkelbereich von 90° oder 120° überspannt, verteilt werden.

Das Ausführungsbeispiel aus Fig. 12A und 12B zeigt im Gegensatz zu Fig. 1A keine parallel zur aktuierten Komponente ausgerichteten Spulen. Stattdessen ist eine alternative Konfiguration eines Axialflussmotors, aufgebaut mit torodial um eine Magnetrückflusskomponente gewickelten Spulen, abgebildet. Dies soll verdeutlichen, dass die vorgestellten Methoden zur Steuerbarkeitsanalyse und entsprechenden Auslegung von konkreten elektrischen Baugruppen mit sechsdimensionaler Steuerbarkeit bei allen bekannten Motorkonfigurationen, insbesondere Motorkonfigurationen mit einer Axialflusskomponente, angewendet werden können.

Im Folgenden werden noch verschiedene elektrische Systeme beschrieben, die jeweils eine elektrische Baugruppe der beschriebenen Art sowie eine (jeweils nicht explizit in den Abbildungen gezeigte) Steuerschnittstelle umfassen, mittels derer die Baugruppe, insbesondere die stationäre Komponente der Baugruppe, mit einer Steuereinheit, insbesondere der oben genannten Steuereinheit, verbindbar oder verbunden ist.

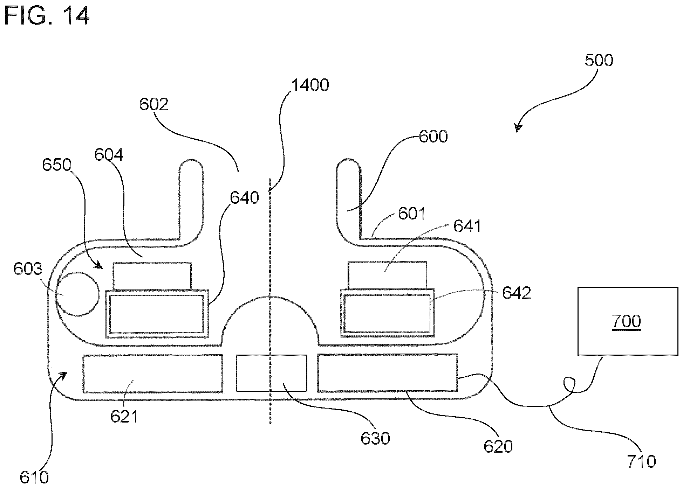

FIG. 14 zeigt ein Herzunterstützungssystem 500 mit einer Blutpumpe 600 (Rotationsfluidpumpe) und einer mit der Blutpumpe 600 mittels einer Driveline 710 verbundene Steuereinheit 700.

Die Blutpumpe 600 umfasst einen Axialflussmotor 610 mit einem Stator 620 (stationäre Komponente) sowie einem magnetisch lagerbaren und zum Fördern von Blut um Rotationsachse 1400 rotierbaren Rotor 640 (aktuierbare Komponente). Der Stator 620 umfasst eine Mehrzahl von Spuleneinheiten 621.

Der Stator 620 ist an einem Gehäuse 601 der Blutpumpe 600 angeordnet, das eine Kavität 604 bildet, in der der Rotor 640 aufgenommen ist. Das Gehäuse 601 umfasst einen Fluideinlass 602 und einen Fluidauslass 603, die mit jeweiligen Blutgefäßen und/oder einem Herzen fluidisch verbindbar sind.

Die Rotationsachse 1400 ist die Hauptachse einer durch die Spuleneinheiten 621 verursachbaren Rotation des Rotors, welche das Fluid antreibt.

Der Rotor 640 umfasst eine Magnetanordnung 642, die mit den von den Spuleneinheiten 621 erzeugten Magnetfeldern wechselwirkt und so den Axialflussmotor 610 bildet.

Der Stator 620 umfasst einen Sensor 630 (Beschleunigungs- oder Kraftsensor), der zum Minimieren von Vibrationen wie oben beschrieben verwendbar ist.

Bei der Blutpumpe 600 handelt es sich um eine implantierbare Blutpumpe (implantierbares VAD), jedoch sind auch wenigstens teilweise extrakorporale Ausführungen denkbar. Die Steuereinheit 700 ist eine extrakorporale Steuereinheit. Die Steuereinheit kann jedoch auch ganz oder teilweise in die Blutpumpe integriert und/oder mit dieser implantierbar sein. Es kann auch vorgesehen sein, dass die Steuereinheit sowohl an der Blutpumpe angeordnete als auch extern/extrakorporal angeordnete Teile umfasst.

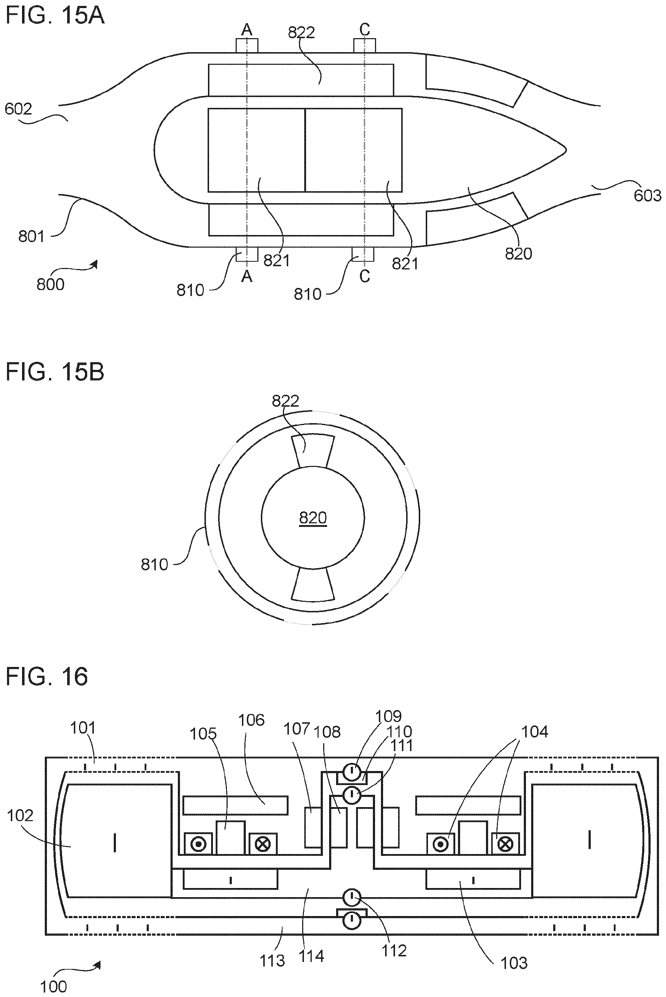

Die Blutpumpe 600 ist im gezeigten Beispiel eine Zentrifugalpumpe, jedoch nicht darauf beschränkt. So zeigen FIG. 15A und FIG. 15B eine als Axialpumpe gebildete Blutpumpe 800. Diese umfasst einen in einem Gehäuse 801 gelagerten Rotor 820 mit Schaufeln 822 und der Magnetanordnung 821. Am Gehäuse 801 ist eine Mehrzahl von Spuleneinheiten 810 angeordnet, die eine erste Gruppe von Spuleneinheiten 810 und eine zweite Gruppe von Spuleneinheiten 810 umfasst, wobei die Spuleneinheiten 810 der ersten Gruppe in einer ersten Ebene A--A angeordnet sind und die Spuleneinheiten 810 der zweiten Gruppe in einer zweiten (zu der ersten Ebene A--A parallelen) Ebene C--C angeordnet sind, so dass zwei parallel angeordnete Statoren gebildet werden. Der Rotor 822 umfasst zwei entsprechende Magnetanordnungen, die zum Wechselwirken mit dem jeweiligen Stator eingerichtet sind. Es können insbesondere sechs oder mehr Spuleneinheiten 810 auf die beiden Ebenen A--A und C--C aufgeteilt werden (beispielsweise in der Kombination 3+3, 3+4 oder 4+4 etc.), um alle sechs Freiheitsgrade Ansteuern und so beispielsweise Schaufelspalte und Schwingungen genau kontrollieren zu können.

Die Spulen können, wie in Fig. 15A abgebildet, wie in einem Radialflussmotor angeordnet sein. Der gezeigte axiale Versatz der Gruppe von Spuleneinheiten 810 gegenüber der Magnetanordnung 821 gibt dem Radialflussmotor zusätzlich einen Axialflussanteil. Bezüglich dieses Axialflussanteils gilt auch die Steuerbarkeit für die in Fig. 13 gezeigten Konfigurationen. Dadurch wird, insbesondere mit einer Verdrehung der Statoren 810 oder der Magnetanordnungen 821 zueinander, eine Steuerbarkeit in sechs Freiheitsgraden mit insgesamt sechs unabhängigen Spuleneinheiten möglich.

Der in FIG. 16 gezeigte Lüfter 100 lässt sich sowohl mit einem erstfehlersicheren Motor als auch mit einer Ausführung mit weniger Phasen realisieren. Der Lüfter 100 umfasst zwei starre Komponenten: Gehäuse 113 und Rotor 114. Der Rotor 114 enthält Schaufeln 102 zum Befördern von Fluid sowie eine Magnetanordnung mit Motormagneten 103. Diese werden von einem Axialflussmotor, gebildet aus Stator 104 mit einem optionalen Eisenkern 105 sowie den Motormagneten 103, angetrieben. Das Fördermedium strömt durch Öffnungen 101 im Gehäuse. Die Rotorposition wird von Rotorpositionssensoren 107 gemessen, welche entweder die Position der Magnete 111 und 112 oder die Position eines optionalen Sensortargets 108 bestimmen. Eine elektronische Steuereinheit 106 steuert den Stator 104 auf Grundlage der Sensordaten an. Grundsätzlich ist die Lagerung ohne die durch die Magnete 109, 111, 112 gebildeten passiven Magnetlager möglich. Diese ermöglichen allerdings einen effizienteren Betrieb durch den Einsatz eines Null-Kraft-Reglers und zusätzliche Notlaufeigenschaften. Ist die aktive Magnetlagerung nicht vollständig einsatzbereit, dann wird das Magnetpaar 109/111 oder 112 den Rotor anziehen, sodass der Rotor in einem Fanglager einseitig Kontakt hat. Das Magnetpaar auf der anderen Seite sorgt dann für eine Kippstabilisierung. Zur Verbesserung der Notlaufeigenschaften kann ein Gleitlagermaterial 110 als Lauffläche eingesetzt werden. Vorteil einer vollständig aktiven Magnetlagerung bei Lüftern oder Pumpen ist zudem, dass die auf den Rotor wirkenden Kräfte gemessen werden können. Die Kräfte können über die Aktorkraft oder auch die Rotorverschiebung, eventuell gravitationskompensiert mittels eines Beschleunigungssensors korrigiert, ermittelt werden. Dadurch können Fluss und insbesondere der Staudruck gemessen werden. Hierdurch ist eine Detektion einer Blockade des Ansaugtrakts oder ein erhöhter Gegendruck durch verdreckte Kühllamellen erkennbar.

Der in Fig. 16 dargestellte Lüfter kann auch mit einem Motor nach Fig. 12A, 12B, 12C oder 13 ausgestattet sein. Der zweiseitige Stator ermöglicht es, passive Magnetkräfte, wie beispielsweise die Anziehungskräfte zwischen Magnetanordnung und Rückflusseisen, statisch auszugleichen. Gleichzeitig kann auf ein separates passives Magnetlager verzichtet werden. Hierdurch sind alle Magnete des Rotors auch Teil der Magnetanordnung 103. Hierdurch wird das Gewicht des Rotor gering gehalten, während durch eine gute magnetische Kopplung zu den Rückflusseisen die Motoren effizient gestaltet werden können. Beide Eigenschaften ermöglichen es, auch schwächere und insbesondere kostengünstigere Ferritmagnete einsetzen zu können.

Ein Reaktionsrad kann als Abwandlung des Lüfters nach FIG. 16 gebildet sein. Hierzu werden lediglich die Schaufeln 102 als Schwungmasse ausgeführt. Um die Erstfehlersicherheit auch auf die Sensorik 107, 108, 111, zu erstrecken, kann diese mehrfach oder überbestimmend ausgeführt werden. Auch hierbei können Versionen mit beidseitigen passiven Magnetlagern, einseitigen Magnetlagern oder ohne passive Magnetlager realisiert werden. Beim Einsatz von passiven Magnetlagern ist die Dämpfung der Resonanzfrequenzen besonders wichtig, da die Dämpfung im Vakuum null beträgt und passiv nur über verlustbehaftete Wirbelströme oder aktiv gedämpft werden kann.

Die elektrische Baugruppe kann als Generator betrieben werden, während gleichzeitig die Lageregelung über dieselben Spuleneinheiten erfolgt. Die in Fig. 14, 15A und 16 gezeigten Pumpsysteme können damit auch zur Stromerzeugung eingesetzt werden.

Mittels einer anmeldungsgemäßen elektrischen Baugruppe kann eine Masse oder ein Messsystem im Raum in bis zu sechs Freiheitsgraden stabilisiert werden. Dies ist nicht nur für die Erzeugung von Antriebsmoment interessant. Die Bewegung einer Masse kann wie beim Reaktionsrad genutzt werden, um Schwingungen eines Gesamtsystems auszugleichen. Hierzu kann ein Beschleunigungssensor am Stator angebracht sein, dessen Sensordaten genutzt werden, um mittels Rückkopplung die Statorbewegungen zu minimieren. Die dem Rotor entsprechende stationäre Komponente kann dazu in einigen Freiheitsgraden blockiert oder zumindest beschränkt sein.

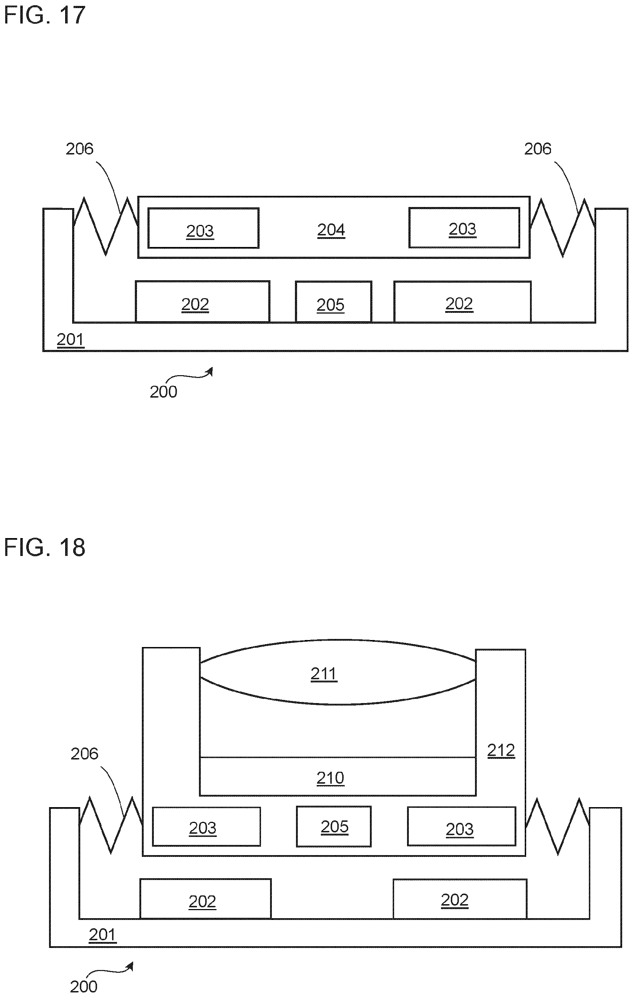

Ein Beispiel zeigt FIG. 17. Die Stabilisationsvorrichtung 200 ist dazu eingerichtet, ein mit Spuleneinheiten des Stators (Statorspulen 202) verbundenes Referenzsystem 201 zu stabilisieren. Hierzu ist eine Reaktionsmasse 204 mit Federn 206 über dem Stator aufgehängt. Durch die Magnete 203 in der Reaktionsmasse kann diese mittels der Statorspulen 202 bewegt werden, um durch actio=reactio eine Kraft auf das Referenzsystem 201 auszuüben, welches mit einem Referenzsensor 205 verbunden ist.

Eine Abwandlung des Beispiels nach FIG. 17 zeigt FIG. 17. Die Stabilisationsvorrichtung 200 ist hier dazu eingerichtet, ein Sensorsystem, insbesondere ein optisches Abbildungssystem umfassend einen Bildsensor 210 und eine Linse 211, zu stabilisieren. Um gleichzeitig die Gesamtmasse für ein mobiles System gering zu halten, wirkt der Stator 202 nicht auf eine Referenzmasse, sondern auf einen Sensorträger 212. Der Referenzsensor 205 ist in diesem Fall über den Sensorträger 212 mit den Motormagneten 203 fest verbunden.

### Liste der Bezugszeichen

100 - Lüfter,
101 - Öffnung,
102, 822 - Schaufeln,
103, 203 - Motormagnete, Magnetanordnung der aktuierbaren Komponente
104, 309, 620 - Stator,
105 - Eisenkern,
106, 700 - Steuereinheit,
107 - Rotorpositionssensor,
108 - Sensortarget,
109, 111, 112, 203 - Magnet,
110 - Gleitlagermaterial,
113, 601, 801 - Gehäuse,
114, 311, 640, 820 - Rotor,
200 - Stabilisationsvorrichtung,
201 - Referenzsystem,
202 - Statorspulen,
204 - Reaktionsmasse,
205 - Referenzsensor,
206 - Feder,
210 - Bildsensor,
211 - Linse,
212 - Sensorträger,
301 - Sensorvektor,
302 - Regler,
303 - Zielvektor,
304 - Aktorenvektor,
305, 405 - Kommutierung,
306 - Phasenstrom-Zielvektor,
307, 407 - Leistungsverstärker,
308, 408 - Phasenstrom,
310 - Magnetfeld,
312 - Rotorposition,
313, 413 - Rotorpositionssensor,
314, 414 - Kommutierungsmatrix,
401 - Freiheitsgrad-Ziel-Stromvektor,
402 - Freiheitsgrad-Strom-Fehler-Vektor,
403 - Individuelle Strom-Regler,
404 - Freiheitsgrad-Spannung-Vektor,
406 - Phasenspannungsvektor,
409 - elektrischer Stator,
410 - magnetischer Stator,
415 - Phasenspannung,
416 - Stromsensoren,
417 - Verknüpfungsmatrix,
418 - Transformation fest zu rotierend,
419 - erfasster Freiheitsgrad-Stromvektor,
420 - magnetflussorientiertes Bezugssystem,
421 - statorfestes Bezugssystem,
422 - Rotor-Dynamik,
423 - Kraft & Moment (tatsächlich),
500 - Herzunterstützungssystem,
600, 800 - Blutpumpe,
602 - Fluideinlass,
603 - Fluidauslass,
604 - Kavität,
610 - Axialflussmotor,
621, 810, 1210 - Spuleneinheit,
630 - Sensor,
642, 1310 - Magnetanordnung,
710 - Driveline,
1100 - elektrische Baugruppe,
1200 - stationäre Komponente,
1211 - Windung,
1300 - aktuierbare Komponente,
1311 - Polpaar,
1400 - Rotationsachse,
2001 - erster Stator,
2002 - zweiter Stator,
2003 - Gruppe ohne Statorverdrehung,
2004 - Gruppe mit Statorverdrehung.

## Patentansprüche

1. Elektrische Baugruppe (1100), umfassend
eine stationäre Komponente (1200), umfassend eine Mehrzahl von Spuleneinheiten (1210), wobei jede der Mehrzahl von Spuleneinheiten (1210) zum Erzeugen eines variablen Magnetfelds ansteuerbar ist,
eine aktuierbare Komponente (1300), die relativ zu der stationären Komponente (1200) beweglich ist und eine Magnetanordnung (1310) umfasst,
wobei jede einzelne der Mehrzahl von Spuleneinheiten (1210) zum Erzeugen des jeweiligen variablen Magnetfelds derart eingerichtet ist, dass durch Wechselwirkung des jeweiligen variablen Magnetfelds mit der Magnetanordnung (1310) Kräfte und/oder Momente bezüglich mehrerer Freiheitsgrade auf die aktuierbare Komponente (1300) wirken,
so dass durch Ansteuern einer beliebigen Kombination von höchstens sechs der Mehrzahl von Spuleneinheiten (1210) eine Position und/oder Bewegung der aktuierbaren Komponente (1300) relativ zu der stationären Komponente (1200) in sechs linear unabhängigen Freiheitsgraden steuerbar ist.

2. Elektrische Baugruppe (1100) nach Anspruch 1, wobei zu jeder vorgegebenen Spuleneinheit (1210) der Mehrzahl von Spuleneinheiten (1210) eine erste Position der aktuierbaren Komponente (1300) und eine zweite Position der aktuierbaren Komponente (1300) existieren,
so dass in der ersten Position durch Wechselwirkung der Magnetanordnung (1310) mit dem mittels der vorgegebenen Spuleneinheit (1210) erzeugbaren variablen Magnetfeld eine Kraft und/oder ein Moment erzeugbar ist, das bezüglich eines ersten Freiheitsgrades der sechs linear unabhängigen Freiheitsgrade auf die aktuierbare Komponente (1300) wirkt und
so dass in der zweiten Position durch Wechselwirkung der Magnetanordnung (1310) mit dem mittels der vorgegebenen Spuleneinheit (1210) erzeugbaren variablen Magnetfeld eine Kraft und/oder ein Moment erzeugbar ist, das bezüglich eines zweiten Freiheitsgrades der sechs linear unabhängigen Freiheitsgrade auf die aktuierbare Komponente (1300) wirkt.

3. Elektrische Baugruppe (1100) nach einem der vorhergehenden Ansprüche, wobei zu jedem vorgegebenen der sechs Freiheitsgrade mindestens eine erste Spuleneinheit (1210) der Mehrzahl von Spuleneinheiten (1210) und eine zweite Spuleneinheit (1210) der Mehrzahl von Spuleneinheiten (1210) existieren,
so dass durch Wechselwirkung der Magnetanordnung (1310) mit dem mittels jeder einzelnen der ersten Spuleneinheit (1210) und der zweiten Spuleneinheit (1210) erzeugbaren variablen Magnetfeld eine Kraft und/oder ein Moment erzeugbar ist, das bezüglich des vorgegebenen Freiheitsgrades auf die aktuierbare Komponente (1300) wirkt.

4. Elektrische Baugruppe (1100) nach einem der vorhergehenden Ansprüche, wobei jede der Mehrzahl von Spuleneinheiten (1210) so verschaltet ist, dass sie unabhängig von jeder anderen der Mehrzahl von Spuleneinheiten (1210) zum Erzeugen des jeweiligen variablen Magnetfelds ansteuerbar ist.

5. Elektrische Baugruppe (1100) nach einem der vorhergehenden Ansprüche, wobei die stationäre Komponente (1200) und die aktuierbare Komponente (1300) einen Axialflussmotor oder eine Axialfluss-Komponente eines Elektromotors bilden, wobei jede der Mehrzahl von Spuleneinheiten (1210) einer Motorphase des Axialflussmotors bzw. der Axialfluss-Komponente entspricht.

6. Elektrische Baugruppe (1100) nach Anspruch 5, wobei die Mehrzahl von Spuleneinheiten (1210) eine erste Gruppe von Spuleneinheiten (1210) und eine zweite Gruppe von Spuleneinheiten (1210) umfasst, wobei die Spuleneinheiten (1210) der ersten Gruppe zum Bilden eines ersten Axialflussmotor-Stators in einer ersten Ebene angeordnet sind und die Spuleneinheiten (1210) der zweiten Gruppe zum Bilden eines zweiten Axialflussmotor-Stators in einer zweiten Ebene angeordnet sind.

7. Elektrische Baugruppe (1100) nach Anspruch 6, **gekennzeichnet durch** einen konstruktiv bedingten Versatz zwischen jeweiligen Wirkrichtungen und/oder Spulenachsen der Spuleneinheiten des ersten Axialflussmotor-Stators und des zweiten Axialflussmotor-Stators, insbesondere durch einen rotatorischen Versatz einer Anordnung der Spuleneinheiten des ersten Axialflussmotor-Stators gegenüber einer Anordnung der Spuleneinheiten des zweiten Axialflussmotor-Stators und/oder durch einen rotatorischen Versatz jeweiliger Polpaare zweier Magnetanordnungen gegeneinander und/oder durch eine schiefwinklige Magnetisierung einer gemeinsamen Magnetanordnung.

8. Elektrische Baugruppe (1100) nach einem der vorhergehenden Ansprüche, wobei die Mehrzahl von Spuleneinheiten (1210) höchstens acht, insbesondere genau sieben oder genau sechs, Spuleneinheiten (1210) umfasst, wobei vorzugsweise die Mehrzahl von Spuleneinheiten (1210) genau sieben Spuleneinheiten (1210) und die Magnetanordnung (1310) der aktuierbaren Komponente (1300) genau zwei, fünf oder neun Polpaare umfasst oder wobei vorzugsweise die Mehrzahl von Spuleneinheiten (1210) genau acht Spuleneinheiten (1210) und die Magnetanordnung (1310) der aktuierbaren Komponente (1300) genau zwei oder sechs Polpaare umfasst.

9. Elektrische Baugruppe (1100) nach Anspruch 6 oder 7, wobei der erste Axialflussmotor-Stator und der zweite Axialflussmotor-Stator jeweils genau drei Spuleneinheiten (1210) aufweisen und die Magnetanordnung ein, drei oder fünf Polpaare aufweist.

10. Elektrische Baugruppe (1100) nach einem der vorhergehenden Ansprüche, wobei jeweilige magnetische Komponenten, insbesondere Spulen und/oder Statorzähne, mindestens zweier Spuleneinheiten (1210) der Mehrzahl von Spuleneinheiten (1210) zueinander asymmetrisch, insbesondere nicht äquidistant, angeordnet und/oder unterschiedlich dimensioniert und/oder unterschiedlich geformt sind.

11. Steuerverfahren für eine elektrische Baugruppe (1100) nach einem der vorhergehenden Ansprüche, umfassend:
Bestimmen einer Mehrzahl von Steuervorgaben zum Ansteuern jeder der Mehrzahl von Spuleneinheiten (1210),
Steuern und/oder Regeln einer Position und/oder Bewegung der aktuierbaren Komponente (1300) relativ zu der stationären Komponente (1200) durch Beaufschlagen der Mehrzahl von Spuleneinheiten (1210) mit einem Signal gemäß der bestimmten Mehrzahl von Steuervorgaben.

12. Steuerverfahren nach Anspruch 11, wobei die Mehrzahl von Steuervorgaben als Steuervorgabenvektor unter Verwendung einer Matrixmultiplikation einer Kommutierungsmatrix, insbesondere einer von der Position der aktuierbaren Komponente (1300) abhängigen Kommutierungsmatrix, mit einem Kraft-Moment-Vektor bestimmt wird, wobei der Kraft-Moment-Vektor ein Vektor mit Kraft- und/oder Momenteinträgen ist,
wobei die Kommutierungsmatrix auf Grundlage einer Verknüpfungsmatrix unter Berücksichtigung mindestens eines Optimierungskriteriums, beispielsweise einer Minimierung einer Verlustleistung und/oder einer Maximierung einer Steuerbarkeit, bestimmt wird, wobei die Verknüpfungsmatrix so definiert ist, dass sich der Kraft-Moment-Vektor durch Matrixmultiplikation aus der Verknüpfungsmatrix und dem Steuervorgabenvektor ergibt.

13. Steuerverfahren nach einem der Ansprüche 11 bis 12, umfassend:
Bestimmen einer Querabhängigkeit des Steuerns und/oder Regelns der Position und/oder Bewegung der aktuierbaren Komponente (1300) relativ zu der stationären Komponente (1200) bezüglich eines ersten Freiheitsgrades von der Position und/oder Bewegung der aktuierbaren Komponente (1300) relativ zu der stationären Komponente (1200) bezüglich eines zweiten Freiheitsgrades,
Bestimmen und/oder Anpassen der Steuervorgaben unter Berücksichtigung der bestimmten Querabhängigkeit, insbesondere zum Minimieren der Querabhängigkeit.

14. Steuerverfahren nach einem der Ansprüche 11 bis 13, umfassend ein Minimieren einer Unwucht der aktuierbaren Komponente (1300) bezüglich einer Rotation relativ zu der stationären Komponente (1200) um eine Rotationsachse durch dynamisches Festlegen und/oder Anpassen einer Position und/oder Orientierung der Rotationsachse in Bezug auf die aktuierbare Komponente (1300), insbesondere basierend auf mittels eines an der aktuierbaren Komponente (1300) und/oder stationären Komponente (1200) angeordneten Beschleunigungssensors und/oder Kraftsensors erfassten Sensordaten und/oder basierend auf einer Minimierung mindestens eines der Einträge des Kraft-Moment-Vektors.

15. Steuerverfahren nach einem der Ansprüche 11 bis 14, umfassend:
Erfassen mindestens einer Messgröße, die ein Erfassen eines Fehlerzustands der elektrischen Baugruppe (1100) ermöglicht, wobei der Fehlerzustand insbesondere ein Ausfallen mindestens einer ersten Spuleneinheit (1210) der Mehrzahl von Spuleneinheiten (1210) und/oder eine nicht vorgesehene elektrische Verbindung mindestens zweier Spuleneinheiten (1210) der Mehrzahl von Spuleneinheiten (1210) umfasst, und
bei einem Erfassen des Fehlerzustands: Anpassen einer Vorschrift zum Bestimmen der Mehrzahl von Steuervorgaben derart, dass das Steuern und/oder Regeln der Position und/oder Bewegung der aktuierbaren Komponente (1300) relativ zu der stationären Komponente (1200) unter Verwendung der Mehrzahl von Spuleneinheiten (1210) unter Berücksichtigung des Fehlerzustands, insbesondere ohne die erste Spuleneinheit (1210), erfolgt.

16. Steuerverfahren nach Anspruch 15, wobei nach Anpassen der Vorschrift zum Bestimmen der Mehrzahl von Steuervorgaben durch Ansteuern einer beliebigen Kombination von höchstens fünf der Mehrzahl von Spuleneinheiten (1210) eine Position und/oder Bewegung der aktuierbaren Komponente (1300) relativ zu der stationären Komponente (1200) unter Ausnutzung einer Trägheit der aktuierbaren Komponente (1300) in einem bewegten, insbesondere rotierenden, Zustand der aktuierbaren Komponente (1300) in den sechs linear unabhängigen Freiheitsgraden steuerbar ist.

17. Steuereinheit (700), eingerichtet zum Ansteuern der elektrischen Baugruppe (1100) nach einem der Ansprüche 1 bis 10 gemäß dem Steuerverfahren nach einem der Ansprüche 11 bis 16.

18. Elektrisches System, umfassend
die elektrische Baugruppe (1100) nach einem der Ansprüche 1 bis 10 und
eine Steuerschnittstelle mittels derer die stationäre Komponente (1200) mit einer Steuereinheit (700), insbesondere der Steuereinheit (700) nach Anspruch 17, verbindbar oder verbunden ist,
wobei das elektrische System insbesondere als Pumpsystem für ein gasförmiges Fördermedium und/oder flüssiges Fördermedium ausgebildet ist.

19. Elektrisches System nach Anspruch 18, ausgebildet als Herzunterstützungssystem und/oder Fontanzirkulations-Unterstützungssystem, wobei die stationäre Komponente (1200) ein Stator einer Rotationsfluidpumpe zum Fördern von Blut ist, wobei die aktuierbare Komponente (1300) ein Rotor der Rotationsfluidpumpe ist,
wobei der Rotor relativ zu dem Stator vorzugsweise in mindestens einem Freiheitsgrad, insbesondere in allen sechs linear unabhängigen Freiheitsgraden, magnetisch gelagert oder lagerbar, insbesondere aktiv magnetisch gelagert oder lagerbar, ist.
